# EUROPEAN PATENT APPLICATION

(11) **EP 4 835 449 A1**
(43) Date of publication of application: **04.11.2026**
(21) Application number: 24910385.4
(22) Date of filing: 15.11.2024
(51) Int. Cl.: G01N 21/76

(54) **CHEMILUMINESCENCE ANALYZER**

(30) Priority: 29.12.2023 CN 202311855738
(71) Applicant: Shenzhen Mindray Bio-Medical Electronics Co., Ltd., Shenzhen, Guangdong 518057 (CN)
(72) Inventor: LIU, Kun, Shenzhen, Guangdong 518057 (CN); CHEN, Pingzhang, Shenzhen, Guangdong 518057 (CN); CHEN, Puguang, Shenzhen, Guangdong 518057 (CN); LI, Ke, Shenzhen, Guangdong 518057 (CN); LIAO, Zhiwei, Shenzhen, Guangdong 518057 (CN); CHEN, Liu, Shenzhen, Guangdong 518057 (CN)
(74) Representative: KIPA AB
(86) International application number: PCT/CN2024/132311
(87) International publication number: WO 2025/139450

(57) **Abstract**

The present disclosure relates to a chemiluminescence analyser. The chemiluminescence analyser includes: a sample preparation apparatus, which is configured to mix a blood sample to be tested and a reaction reagent in a reaction vessel, so as to obtain a sample solution to be tested; a substrate providing apparatus, which is configured to provide a luminescent substrate into the reaction vessel containing said sample solution, so as to carry out a chemiluminescence reaction; a photometric apparatus, which at least comprises a photometer and is configured to detect a luminescent signal generated in the chemiluminescence reaction; and a data processing apparatus, which is configured to obtain, on the basis of the luminescent signal and a preset calibration model, a test result of a substance to be tested in said blood sample. A first linear measurement range of the photometer is [A1, A2] photons per second, wherein A1 is less than or equal to 2000, and A2 is greater than or equal to 10⁸. A second linear measurement range of the luminescent substrate is [B1, B2] photons per second, wherein B1 is less than or equal to 3000, and B2 is greater than or equal to 10⁸. By means of the embodiments of the present disclosure, a chemiluminescence analyser having a high sensitivity and a high upper limit of linear measurement can be obtained.

## Description

### TECHNICAL FIELD

The present disclosure relates to the field of chemiluminescence detection, and in particular relates to a chemiluminescence analyser.

### BACKGROUND

Chemiluminescence immunoassay (CLIA) is an immunoassay technology that combines a chemiluminescence system with immunoreactions to detect antigens, antibodies, hormones, fatty acids, vitamins, drugs, and the like. Chemiluminescence refers to a phenomenon in which specific substances absorb part of chemical energy during a chemical reaction to reach an excited state, and when these substances return to the ground state, the energy is released in the form of photons, resulting in light emission. Chemiluminescence immunoassay not only possesses the high sensitivity of chemiluminescence but also features simple operation and rapid reaction of enzymelinked immunosorbent assay, making it easy to standardize experimental operations. CLIA has now been widely used in biology, medical research, and clinical diagnosis.

However, for a chemiluminescence analyser based on chemiluminescence immunoassay, it is difficult to take both detection sensitivity and a linear detection range into account, particularly for an analyte having a large range, such as human chorionic gonadotropin (HCG).

Human chorionic gonadotropin (HCG) is a glycoprotein mainly secreted by placental trophoblast cells. HCG is composed of an α subunit and a β subunit, containing 237 amino acids, with a molecular weight of about 38,000 Daltons. Each subunit is composed of a single polypeptide, wherein the α subunit is composed of 92 amino acids, with a molecular weight of about 14,900 Daltons; and the β subunit is composed of 145 amino acids, with a molecular weight of about 23,000 Daltons. In the structure of HCG, proteins accounts for 70%, and carbohydrates accounts for 30%.

HCG may be found in urine, blood, and placenta, and its molecular forms vary among different biological matrices. HCG mainly exists in blood in its intact form. The structure of the α subunit is similar to those of α subunits of follicle-stimulating hormone, luteinizing hormone, and thyroid-stimulating hormone secreted by the pituitary gland. The structures of β subunits are different from one another, and the differences in the β subunits cause their respective immunological and biological specificities.

The main function of human chorionic gonadotropin (HCG) is to stimulate the corpus luteum to maintain continuous secretion of estrogen and progesterone, so as to promote the formation of uterine decidua and the growth and maturation of the placenta. HCG is produced by trophoblast transitional cells and syncytial cells and is regulated by multiple factors including autocrine and paracrine signalling. In women with normal pregnancy, HCG starts to be produced 2 to 8 days after conception. In early pregnancy, the HCG content approximately doubles every 31-48 hours, peaking around the 11th week of pregnancy, then slowly decreases in concentration until the 18th to 20th week, and then remains relatively constant. The detection of HCG is of great significance for early pregnancy diagnosis and pregnancy monitoring. In early pregnancy, an increase in HCG by 35-50% within two days indicates possible ectopic pregnancy, and an abnormally low HCG level or an abnormally sharp decrease in the HCG level indicates a high risk of ectopic pregnancy. On the other hand, in the second trimester of pregnancy, an increase in the HCG level indicates potential adverse reactions to pregnancy, possibly pregnancy-induced hypertension, fetal growth restriction, and the like.

HCG has a relatively wide concentration distribution in normal non-pregnant women, pregnant women, and individuals with trophoblastic cell diseases. For pregnant individuals, their HCG may increase from several hundred mIU/mL in very early pregnancy to a maximum of about 200,000 mIU/mL, and then decrease to a level below about 10,000 mIU/mL. For patients with trophoblastic cell diseases (including choriocarcinoma), their HCG concentration may be as high as 1000000 mIU/mL, or even higher. For non-pregnant women, their HCG concentrations are typically below 5 mIU/mL, and in some cases even as low as 0.5 mIU/mL.

That is, HCG is secreted by placental trophoblast cells, and its content in non-pregnant individuals is very low, less than 5 mIU/ml. Therefore, high detection sensitivity is a key performance for ensuring the clinical use of HCG test results to assist in determining pregnancy. Meanwhile, in a pregnancy monitoring scenario, it is necessary to continuously measure the HCG concentration in early pregnancy (about 200,000 mIU/ml). Therefore, a wide linear detection range is a key performance for ensuring the clinical use of HCG test results for monitoring pregnancy status.

Therefore, for a chemiluminescence analyser, in an HCG test scenario, a linear range that needs to be accurately detected spans 6 to 7 orders of magnitude. However, the current chemiluminescence analysers and their accompanying reagents have an upper linear detection limit of no more than 15,000 mIU/mL, which is far from sufficient to cover conventional pregnancy testing scenarios.

### SUMMARY

Based on this background, an object of the present disclosure is to provide a chemiluminescence analyser based on chemiluminescence immunoassay and having a wide linear detection range, which can take both sensitivity and an upper limit of linear detection into account. In particular, for a sample having a high concentration of an analyte (for example HCG or β-HCG), dilution and retesting are not required, thereby improving efficiency and saving reagent costs.

To achieve the above objectives, a first aspect of the present disclosure provides a chemiluminescence analyser, including:
a sample preparation device, configured to cause a blood sample to be tested to be mixed with reaction reagents in a reaction vessel, so that an analyte in the blood sample to be tested reacts with the reaction reagents to obtain a sample solution to be tested, wherein the reaction reagents comprise a capture reagent and a detection reagent labeled with a label;
a substrate supply device, configured to supply a luminescent substrate into the reaction vessel containing the sample solution to be tested, so that the label in the sample solution to be tested undergoes a chemiluminescent reaction with the luminescent substrate;
a photometric device, at least comprising a photometer configured to detect a luminescence signal generated in the chemiluminescent reaction;
a data processing device, configured to obtain a test result of the analyte in the blood sample to be tested based on the luminescence signal and a preset calibration model; and
wherein a first linear detection range of the photometer is [A1, A2] photon counts per second, the first linear detection range represents a range of photon counts outputted by the photometer, and within the first linear detection range, an intensity of the luminescence signal generated in the chemiluminescent reaction is in a linear relationship with photon counts outputted by the photometer, wherein A1 is less than or equal to 2000 and A2 is greater than or equal to 10⁸; a second linear detection range of the luminescent substrate is [B1, B2] photon counts per second, the second linear detection range represents a range of the intensity of the luminescence signal generated in the chemiluminescent reaction, and within the second linear detection range, a number of labels participating in the chemiluminescent reaction is in a linear relationship with the intensity of the luminescence signal generated in the chemiluminescent reaction, wherein B1 is less than or equal to 3000 and B2 is greater than or equal to 10⁸.

A second aspect of the present disclosure provides another chemiluminescence analyser, including:
a sample preparation device, configured to cause a blood sample to be tested to be mixed with reaction reagents in a reaction vessel, so that an analyte in the blood sample to be tested reacts with the reaction reagents to obtain a sample solution to be tested, wherein the reaction reagents comprise a capture reagent and a detection reagent labeled with a label;
a substrate supply device, configured to supply a luminescent substrate into the reaction vessel containing the sample solution to be tested, so that the label in the sample solution to be tested undergoes a chemiluminescent reaction with the luminescent substrate;
a photometric device, at least comprising a photometer configured to detect a luminescence signal generated in the chemiluminescent reaction;
a data processing device, configured to obtain a test result of the analyte in the blood sample to be tested based on the luminescence signal and a preset calibration model; and
wherein a first linear detection range of the photometer is [A1, A2] photon counts per second, the first linear detection range represents a range of photon counts outputted by the photometer, and within the first linear detection range, an intensity of the luminescence signal generated in the chemiluminescent reaction is in a linear relationship with photon counts outputted by the photometer, wherein a ratio (A2/A1) of A2 to A1 is greater than or equal to 30000; a second linear detection range of the luminescent substrate is [B1, B2] photon counts per second, the second linear detection range represents a range of the intensity of the luminescence signal generated in the chemiluminescent reaction, and within the second linear detection range, a number of labels participating in the chemiluminescent reaction is in a linear relationship with the intensity of the luminescence signal generated in the chemiluminescent reaction, wherein a ratio (B2/B1) of B2 to B1 is greater than or equal to 30000.

A third aspect of the present disclosure provides yet another chemiluminescence analyser, including:
a sample preparation device, configured to cause a blood sample to be tested to be mixed with a reaction reagent in a reaction vessel, so that an analyte in the blood sample to be tested reacts with the reaction reagent to obtain a sample solution to be tested;
a substrate providing device, configured to supply a luminescent substrate into the reaction vessel containing the sample solution to be tested, so that the sample solution to be tested undergoes a chemiluminescent reaction with the luminescent substrate;
a photometric device, at least comprising a photometer configured to detect a luminescence signal generated in the chemiluminescent reaction;
a data processing device, configured to obtain a test result of the analyte in the blood sample to be tested based on the luminescence signal and a preset calibration model; and
wherein the photometer comprises a receiving component configured to receive the optical signal generated in the chemiluminescent reaction and convert the optical signal into a corresponding electrical signal, and a processing component configured to be electrically connected to the receiving component and receive the electrical signal from the receiving component, wherein the processing component comprises a first photon counting module configured to detect a number of pulses of the electrical signal by using a pulse recognition method to obtain a first photon counting result, and a second photon counting module configured to process the electrical signal to obtain a parameter characterizing a number of photons in the optical signal, and obtain a second photon counting result based on the parameter characterizing the number of photons in the optical signal and a preset calibration function, wherein the preset calibration function represents a mapping relationship between the parameter characterizing the number of photons in the optical signal and a photon counting result, and the processing component is further configured to obtain a photon count outputted by the photometer based on the first photon counting result and the second photon counting result, and outputted the photon count; and
a second linear detection range of the luminescent substrate is [B1, B2] photon counts per second, the second linear detection range represents a range of the intensity of the luminescence signal generated in the chemiluminescent reaction, and within the second linear detection range, a number of the labels participating in the chemiluminescent reaction is in a linear relationship with the intensity of the luminescence signal generated in the chemiluminescent reaction, wherein a ratio (B2/B1) of B2 to B1 is greater than or equal to 30000 or B1 is less than or equal to 3000 and B2 is greater than or equal to 10⁸.

A fourth aspect of the present disclosure provides still another chemiluminescence analyser, including:
a sample preparation device, configured to cause a blood sample to be tested to be mixed with a reaction reagent in a reaction vessel, so that an analyte in the blood sample to be tested reacts with the reaction reagent to obtain a sample solution to be tested;
a substrate providing device, configured to supply a luminescent substrate into the reaction vessel containing the sample solution to be tested, so that the sample solution to be tested undergoes a chemiluminescent reaction with the luminescent substrate;
a photometric device, at least comprising a photometer configured to detect a luminescence signal generated in the chemiluminescent reaction;
a data processing device, configured to obtain a test result of the analyte in the blood sample to be tested based on the luminescence signal and a preset calibration model; and
wherein a first linear detection range of the photometer is [A1, A2] photon counts per second, the first linear detection range represents a range of photon counts outputted by the photometer, and within the first linear detection range, an intensity of the luminescence signal generated in the chemiluminescent reaction is in a linear relationship with photon counts outputted by the photometer, wherein a ratio (A2/A1) of A2 to A1 is greater than or equal to 30000 or A1 is less than or equal to 2000 and A2 is greater than or equal to 10⁸, and the luminescent substrate comprises a chemiluminescent substrate and a chemiluminescence enhancer, the chemiluminescence enhancer comprises a fluorescent agent and a surfactant, and the fluorescent agent comprises one or more carboxyfluoresceins.

In the chemiluminescence analyser provided in each aspect of the present disclosure, a high detection sensitivity and a high upper limit of linear detection can be achieved through the coordinated design of the photometer and the luminescent substrate, that is, the linear detection range of a single detection of the chemiluminescence analyser according to the present disclosure is broadened.

### BRIEF DESCRIPTION OF THE DRAWINGS

The present disclosure will be more clearly explained below with reference to the embodiments and the accompanying drawings. The above and other advantages will become apparent to those of ordinary skill in the art from the detailed description of the embodiments of the present disclosure. The accompanying drawings are only for illustrating preferred embodiments and should not be considered as limiting the present disclosure. In the accompanying drawings:
FIG. 1 shows a schematic block diagram of a chemiluminescence analyser provided by an example of the present disclosure.
FIG. 2 and FIG. 3 show calibration curves of a chemiluminescence analyser provided by an example of the present disclosure.
FIG. 4 shows a schematic structural diagram of a chemiluminescence analyser provided by an example of the present disclosure.
FIG. 5 to FIG. 15 show schematic block diagrams of photometers provided by different examples of the present disclosure.
FIG. 16A to FIG. 16D show the linear fitted curves of the luminescence values of the chemiluminescent substrate solutions 1-1 to 1-4 of Example 1 in an alkaline phosphatase system.
FIG. 17A to FIG. 17D show the linear fitted curves of the luminescence values of the chemiluminescent substrate solutions 2-1 to 2-4 of Example 2 in an alkaline phosphatase system.
FIG. 18A to FIG. 18D show the linear fitted curves of the luminescence values of the chemiluminescent substrate solutions 3-1 to 3-4 of Example 3 in an alkaline phosphatase system.
FIG. 19A to FIG. 19D show the linear fitted curves of the luminescence values of the chemiluminescent substrate solutions 4-1 to 4-4 of Example 4 in an alkaline phosphatase system.
FIG. 20A to FIG. 20D show the linear fitted curves of the luminescence values of the chemiluminescent substrate solutions 5-1 to 5-4 of Example 5 in an alkaline phosphatase system.

### DESCRIPTION OF THE PREFERRED EMBODIMENTS

The embodiments of the present disclosure will be described below clearly and completely with reference to the accompanying drawings. Obviously, the embodiments described are merely some, rather than all, of the embodiments of the present disclosure. Based on the embodiments in the present disclosure, all the other embodiments that would have been obtained by those of ordinary skill in the art without any inventive effort shall fall within the scope of protection of the present disclosure.

It should be noted that the term "first/second/third" in the embodiments of the present disclosure is only used to distinguish similar objects, and does not represent a specific order for the objects. It can be understood that "first/second/third" may be interchanged for specific order or chronological order when allowed.

It can be understood by those skilled in the art that unless otherwise defined, all terms (including technical terms and scientific terms) used herein have the same meanings as those generally understood by those of ordinary skill in the art to which the present disclosure belongs.

As mentioned in the Background Art, certain detection items, such as the HCG test item has a relatively high requirement on an upper limit of linear measurement, whereas the prior arts fail to satisfy the demands of both a high upper linear limit and high detection sensitivity simultaneously.

On this basis, examples of the present disclosure propose to improve the upper limit of linear detection and detection sensitivity by means of the coordinated design of a photometer and a luminescent substrate.

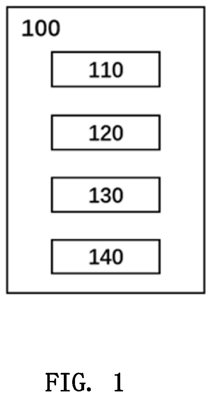

As shown in FIG. 1, an embodiment of the present disclosure provides a chemiluminescence analyser 100, including a sample preparation device 110, a substrate providing device 120, a photometric device 130, and a data processing device 140.

The sample preparation device 110 is configured to cause a blood sample to be tested to be mixed with reaction reagents in a reaction vessel, so that an analyte in the blood sample to be tested reacts with reaction reagents to obtain a sample solution to be tested, wherein the reaction reagents include a capture reagent and a detection reagent labeled with a label. In some embodiments, the capture reagent may be a magnetic bead reagent for capturing an analyte, and the detection reagent labeled with the label may be a detection reagent labelled with an enzyme label, for example alkaline phosphatase.

The substrate providing device 120 is configured to supply a luminescent substrate into the reaction vessel containing the sample solution to be tested, so that the label in the sample solution to be tested undergoes a chemiluminescent reaction with the luminescent substrate.

The photometric device 130 at least includes a photometer configured to detect a luminescence signal generated in the chemiluminescent reaction.

The data processing device 140 is configured to obtain a test result, for example, a concentration, of the analyte in the blood sample to be tested based on the luminescence signal and a preset calibration model.

According to a first embodiment of the present disclosure, a first linear detection range of the photometer is [A1, A2] photon counts per second, the first linear detection range represents a range of photon counts outputted by the photometer, and within the first linear detection range, an intensity of the luminescence signal generated in the chemiluminescent reaction is in a linear relationship with photon counts outputted by the photometer, wherein A1 is less than or equal to 2000 and A2 is greater than or equal to 10⁸. A second linear detection range of the luminescent substrate is [B1, B2] photon counts per second, the second linear detection range represents a range of the intensity of the luminescence signal generated in the chemiluminescent reaction, and within the second linear detection range, a number of labels participating in the chemiluminescent reaction is in a linear relationship with the intensity of the luminescence signal generated in the chemiluminescent reaction, wherein B1 is less than or equal to 3000 and B2 is greater than or equal to 10⁸.

According to a second embodiment of the present disclosure, a first linear detection range of the photometer is [A1, A2] photon counts per second, the first linear detection range represents a range of photon counts outputted by the photometer, and within the first linear detection range, an intensity of the luminescence signal generated in the chemiluminescent reaction is in a linear relationship with photon counts outputted by the photometer, wherein a ratio (A2/A1) of A2 to A1 is greater than or equal to 30000; a second linear detection range of the luminescent substrate is [B1, B2] photon counts per second, the second linear detection range represents a range of the intensity of the luminescence signal generated in the chemiluminescent reaction, and within the second linear detection range, a number of labels participating in the chemiluminescent reaction is in a linear relationship with the intensity of the luminescence signal generated in the chemiluminescent reaction, wherein a ratio (B2/B1) of B2 to B1 is greater than or equal to 30000.

Herein, by designing the photometer and the luminescent substrate as according to the first embodiment of the present disclosure or according to the first embodiment of the present disclosure, a chemiluminescence analyser having a wide linear detection range can be obtained, and in particular, at least a chemiluminescence analyser having a linear detection range of [3000, 10⁸] photon counts per second can be obtained.

In the embodiments of the present disclosure, the linear detection range refers to a linear detection range of a single detection, that is, a linear detection range that can be achieved in one detection of one sample.

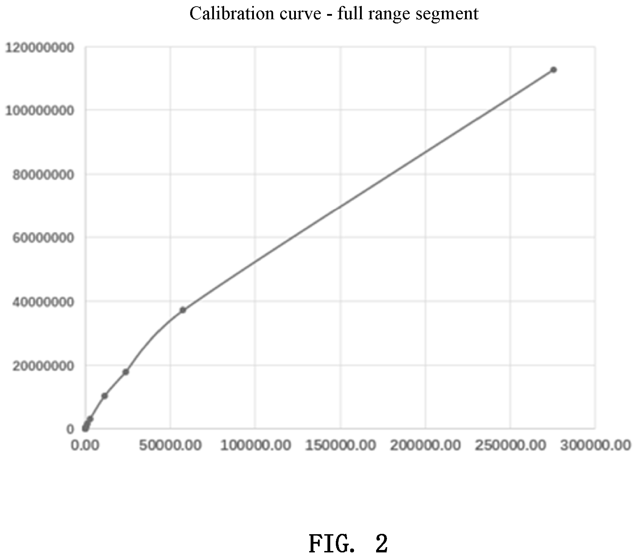

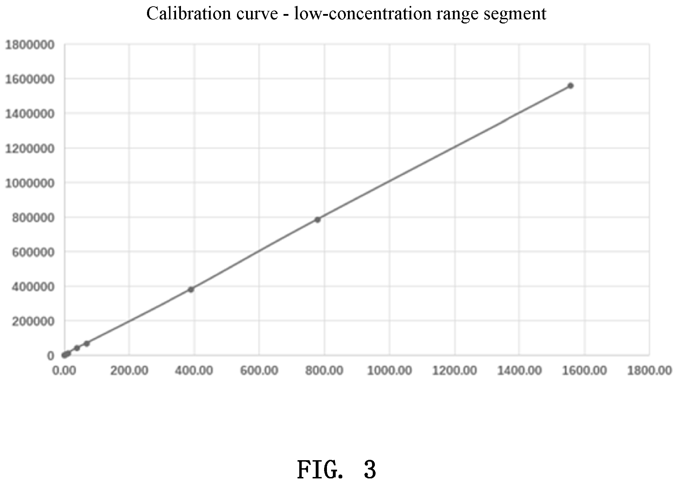

For example, for the HCG test item, the chemiluminescence analyser according to the first embodiment and second embodiment of the present disclosure can accurately and linearly detect HCG as low as 2 mIU/ml and as high as 200,000 mIU/ml, without requiring dilution and retesting. That is, HCG having a concentration between 2 and 200000 mIU/ml can be accurately obtained by one detection, thereby satisfying the main requirements for clinically using HCG to assist in determining whether pregnancy exists and for monitoring a pregnancy state, avoiding dilution and retesting when the HCG concentration of a sample exceeds the upper detection limit, greatly improving the work efficiency of a clinical laboratory, and saving costs. As shown in FIG. 2 and FIG. 3, for the HCG test item, within an HCG concentration range of 2 to 200000 mIU/ml, the calibration model, or the calibration curve, has good linearity in a low-HCG-concentration range, and also has good linearity within the entire HCG concentration range.

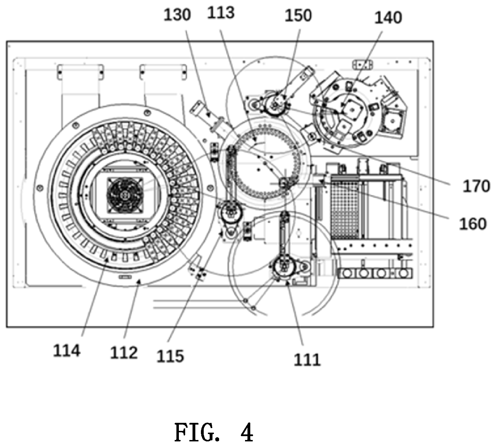

FIG. 4 shows a schematic structural diagram of a chemiluminescence analyser 100 provided by an embodiment of the present disclosure. As shown in FIG. 4, the sample preparation device 110 includes a sample supply unit 111, a reagent supply unit 112, and a reaction incubation unit 113; the sample supply unit 111 is configured to aspirate the blood sample to be tested and supply the blood sample to be tested into the reaction vessel, and the reagent supply unit 112 is configured to supply the capture reagent and the detection reagent labelled with the label into the reaction vessel. The reaction incubation unit 113 is configured to provide a place for reaction and incubation for the reaction vessel containing the blood sample to be tested and the reaction reagents, so that the blood sample to be tested and the reaction reagents in the reaction vessel form the sample solution to be tested.

The chemiluminescence analyser 100 further includes a magnetic separation device 140 and a transfer device 150; the magnetic separation device 140 is configured to perform a magnetic separation operation on the reaction vessel containing the sample solution to be tested, the substrate supply device 120 (not shown) is configured to supply the luminescent substrate into the reaction vessel in which the magnetic separation operation has been completed, and the transfer device 150 is configured to transfer the reaction vessel in which the magnetic separation operation has been completed from the magnetic separation device 140 to the reaction incubation unit 113. The reaction vessel after incubation is transferred to the photometric device 130 for photometry.

In some embodiments, the chemiluminescence analyser 100 further includes a sample processing system not shown, which is configured to convey a test tube containing a blood sample to be tested to a sample aspiration position and convey a test tube on which sample aspiration has been completed to a recovery area. Specifically, the sample processing system includes a sample loading area and a conveying track; the sample loading area is configured to store a test tube containing a blood sample to be tested placed by a user, and the conveying track is configured to convey the test tube in the sample loading area to the sample aspiration position.

In addition, the sample processing system further includes a scanning module for scanning a sample barcode, so as to implement sample identification and management.

In some embodiments, the sample supply unit 111 may include a sample needle and a drive mechanism, and the drive mechanism is configured to drive the sample needle to move between a sample aspiration position and a sample addition position. The sample needle is configured to perform a sample aspiration operation on a sample in a sample tube located at the sample aspiration position, and inject the aspirated sample into a reaction vessel located at the sample addition position. Here, the sample aspiration position is, for example, an intersection of a movement trajectory of the sample needle and a sample conveying trajectory of the conveying track.

In addition, the sample supply unit 111 further includes a cleaning mechanism for cleaning the sample needle. Further, the sample supply unit 111 further includes a dilution position for placing a reaction vessel in which automatic dilution and pretreatment have been completed, so that the sample needle aspirates a diluted sample or a pretreated sample in the reaction vessel located at the dilution position, to support a corresponding test mode.

In some embodiments, the chemiluminescence analyser further includes a first robotic arm 160 for transferring a reaction vessel, and the first robotic arm is configured to be capable of three-dimensional movement and capable of clamping the reaction vessel. The first robotic arm is configured to be capable of loading a new reaction vessel into the sample addition position, so that the sample needle injects a sample. The first robotic arm is further configured to be capable of discarding an empty reaction vessel. The first robotic arm is further configured to be capable of transferring a reaction vessel among the sample addition position, the reaction incubation unit 113, and the dilution position.

In some embodiments, the reagent supply unit 112 includes a reagent disk 114 and a reagent needle 115; the reagent disk 114 is configured to place reagent containers each containing the capture reagent and the detection reagent labeled with the label, and has a function of refrigerating the reagent containers. The reagent disk 114 is configured to be rotatable, so as to transfer a reagent container required for reagent aspiration to a reagent aspiration position. The reagent needle 115 is configured to aspirate a reagent from the reagent container located at the reagent aspiration position and dispense the aspirated reagent into the reaction vessel located at the sample addition position.

Further, the reagent disk has a mixing mechanism, not shown, for mixing a magnetic bead reagent. The mixing mechanism is configured to implement mixing of the capture reagent by, for example, rotating the reagent container containing the capture reagent, for example the magnetic bead reagent.

Further, the reagent supply unit 112 further includes a cleaning mechanism, not shown, for cleaning the reagent needle.

In some embodiments, the magnetic separation device 140 has a disk-shaped rotatable magnetic separation disk, a magnetic mechanism, and a constant-temperature control mechanism; the magnetic separation disk is configured to drive reaction vessels therein to sequentially pass through the magnetic mechanism to perform a magnetic separation operation, and the constant-temperature control mechanism is configured to maintain a temperature of the magnetic separation disk within a preset range.

In some embodiments, the substrate supply device has a heating mechanism for preheating the luminescent substrate. The substrate supply device is configured to inject the preheated luminescent substrate into a reaction vessel located at a substrate injection position and in which magnetic separation has been completed, wherein the substrate injection position is located on the magnetic separation disk.

In some embodiments, the reaction incubation unit 113 includes a disk-shaped rotatable reaction disk, the photometric device 130 is arranged on an outer peripheral side of the reaction disk, and the reaction vessel after the luminescent substrate is injected is transferred into the reaction disk by the transfer device 150. The reaction disk drives the reaction vessel incubated for a certain period of time to enter the photometric device 130 to complete photometric measurement.

In some embodiments, the transfer device 150 is configured as a second robotic arm, and the second robotic arm is configured to clamp a reaction vessel and transfer the reaction vessel between the reaction disk and the magnetic separation disk. The second robotic arm is configured to be capable of horizontal rotation and capable of performing up-and-down two-dimensional movement.

In some embodiments, the chemiluminescence analyser may further include a mixer 170 arranged between the reaction disk and the magnetic separation disk, and the mixer is configured to perform a mixing operation on a reaction vessel to which a reagent and a sample have been added. The mixer may be configured, for example, as a vortex mixer capable of non-contact mixing, thereby effectively avoiding crosscontamination.

In addition, the chemiluminescence analyser may further include a hardware control system not shown, and software systems respectively running on a hardware board and a PC, so as to control the above devices of the chemiluminescence immunoassay analyser to work cooperatively. The chemiluminescence analyser may further include a mechanical system, not shown, composed of a frame, a housing, and the like, so as to provide support and positioning and provide protection for the above devices.

In some embodiments, the data processing device 140 may include, but is not limited to, a central processing unit (CPU), a micro controller unit (MCU), a fieldprogrammable gate array (FPGA), a digital signal data processing device (DSP) and other devices for interpreting computer instructions and processing data in computer software. For example, the data processing device is used for executing computer application programs in a computer-readable storage medium, so that the chemiluminescence analyser 100 executes a corresponding detection process and analyses, in real time, the luminescence signal detected by the photometric device 130.

A specific detection process of the chemiluminescence analyser is as follows: the sample supply unit 111 aspirates the blood sample to be tested and adds the blood sample to be tested into a reaction vessel; the reagent supply unit 112 aspirates the capture reagent and the detection reagent and adds the capture reagent and the detection reagent into the reaction vessel to which the sample has been added, so as to mix with the sample; then the reaction vessel is placed in the reaction incubation unit 113 for reaction, incubation, and magnetic separation cleaning; then the substrate supply device adds the luminescent substrate into the reaction cup in which reaction, incubation, and magnetic separation cleaning have been completed, for incubation for a period of time; finally, the photometric device 130 detects photons emitted by the analyte in the sample to be tested under the action of the luminescent substrate, so that a concentration level of the analyte is calculated according to the number of detected photons.

In some embodiments, the lower limit B1 of the second linear detection range is less than or equal to 2000. Thus, sensitivity of the chemiluminescence analyser can be further improved, thereby further broadening the linear detection range of a single detection of the chemiluminescence analyser.

In some embodiments, the analyte is human chorionic gonadotropin (HCG). In this case, the chemiluminescence analyser 100 may be designed such that: when a concentration of the human chorionic gonadotropin in the blood sample to be tested is greater than or equal to 200000 mIU/ml, the intensity of the luminescence signal generated in the chemiluminescent reaction is less than or equal to a smaller value of A2 and B2. Thus, an HCG concentration as high as 200000 mIU/ml can be accurately detected.

In some embodiments, the analyte is human chorionic gonadotropin (HCG). In this case, the chemiluminescence analyser 100 may be designed such that: when a concentration of the human chorionic gonadotropin in the blood sample to be tested is less than or equal to 0.5 mIU/ml, the intensity C1 of the luminescence signal generated in the chemiluminescent reaction is greater than or equal to a larger value of A1 and B1. Thus, an HCG concentration as low as 0.5 mIU/ml can be accurately detected.

Further, the chemiluminescence analyser 100 may be designed such that: when a concentration of the human chorionic gonadotropin in the blood sample to be tested is greater than 0.5 mIU/ml and less than or equal to 5 mIU/ml, preferably less than or equal to 2 mIU/ml, the intensity C2 of the luminescence signal generated in the chemiluminescent reaction satisfies: a ratio C2/C1 of C2 to C1 is greater than 2.

In some embodiments, the upper limit B2 of the second linear detection range is greater than the upper limit A2 of the first linear detection range.

In some embodiments, the sample preparation device 110 is further configured to: for each blood sample to be tested containing the human chorionic gonadotropin as the analyte, pre-dilute said blood sample to be tested, for example, by a factor of 2, 5, or 10, before mixing said blood sample to be tested with the reaction reagents. Thus, the retest rate of samples with high HCG concentration can be further reduced.

Further, the calibration model may be obtained by the chemiluminescence analyser 100 using a pre-diluted calibrator. A pre-dilution factor of the blood sample to be tested is preferably equal to a pre-dilution factor of the calibrator.

Here, calibration is performed using the pre-diluted calibrator, that is, the pre-diluted calibrator is directly traced to a standard substance, and a gradient concentration response curve of the pre-diluted calibrator is established, thereby eliminating systematic errors caused by random deviations and matrix effects of the dilution process itself, and improving accuracy of measurement results.

In some embodiments, the calibration model, or the calibration curve, is obtained by multi-point calibration.

In the embodiments of the present disclosure, a multi-point calibration method may be: using a series of samples (greater than or equal to 5 samples) pre-assigned by a supplier of the chemiluminescence analyser, measuring the series of samples on the chemiluminescence analyser to obtain luminescence signal values of the series of samples, establishing a curve of sample concentration vs luminescence signal, and performing 4PLC fit regression on the curve to obtain a fitted equation, namely the calibration model.

It can be understood that the luminescence signal value measured for the blood sample to be tested on the chemiluminescence analyser is substituted into the calibration model, to calculate and obtain a concentration of the analyte in the blood sample to be tested.

In some embodiments, the calibration model is obtained by the following formula $RLU = \frac{{P}_{1} - {P}_{4}}{1 + {\left(\frac{C}{{P}_{2}}\right)}^{{P}_{3}}} + {P}_{4}$ wherein RLU is reactivity, namely the measured luminescence signal values; C is a concentration of the analyte; P1 to P4 are four fitting parameters, wherein P1 represents a luminescence signal corresponding to zero concentration (asymptote), P4 represents a luminescence signal corresponding to maximum concentration (asymptote), P2 represents an inflection point at which a curve slope direction changes, and P3 reflects a slope at P2 (not a true slope).

It can be understood here that the 4PLC calibration model uses at least 5 (number of fitting parameters + 1) points to perform 4PLC regression, and obtains an optimal solution through a numerical iterative process, thereby obtaining four fitting parameters P1, P2, P3, and P4.

In some embodiments of the present disclosure, the calibration model is recalibrated when a reagent lot change occurs, or the calibration model is recalibrated at fixed intervals of days, or the calibration model is recalibrated when instrument quality control failure occurs.

Some embodiments of the luminescent substrate used by the chemiluminescence analyser of the present disclosure are described below, but the present disclosure is not limited thereto.

In some embodiments, the luminescent substrate is designed such that a background of the luminescent substrate is less than 3000, thereby further improving sensitivity of the chemiluminescence analyser.

Alternatively or additionally, the luminescent substrate may be designed such that a signal-to-noise ratio of the luminescent substrate is greater than 40000. Thus, sensitivity of the chemiluminescence analyser can be further improved.

In some embodiments, the luminescent substrate comprises a chemiluminescent substrate and a chemiluminescence enhancer, the chemiluminescence enhancer comprises a fluorescent agent and a surfactant, and the fluorescent agent comprises one or more carboxyfluoresceins. Use of such a luminescent substrate can achieve high sensitivity of the chemiluminescence analyser.

As some implementations, the luminescent substrate may be selected from dioxetane compounds, preferably selected from one or more of AMPPD, CSPD and CDP-STAR, and more preferably is CDP-STAR.

As some implementations, the luminescent substrate may be selected from a chlorinated derivative of AMPPD, fluorescein or a carboxy-substituted derivative thereof, and a water-soluble polymeric quaternary ammonium salt cationic surfactant.

The chlorinated derivative of AMPPD may be, for example, ADP-STAR or CDP-STAR. Alternatively, the fluorescein or carboxy-substituted derivative thereof may be, for example, fluorescein or 5(6)-carboxyfluorescein. Alternatively, the water-soluble polymeric quaternary ammonium salt cationic surfactant may be, for example, poly(vinylbenzyltrimethylammonium chloride).

In some embodiments, the substrate supply device is configured to provide a chemiluminescent substrate solution containing a chemiluminescent substrate, or a luminescent substrate, the chemiluminescent substrate solution including a chemiluminescent substrate, fluorescein, and a water-soluble polymeric quaternary ammonium salt, wherein the chemiluminescent substrate is selected from a chlorinated dioxetane compound with a spiroadamantane substituent.

The chemiluminescent substrate refers to a compound that participates in energy transfer in a chemiluminescent reaction and finally releases energy in the form of emitting photons, and is also known as a chemiluminescent reagent or a luminescent substrate. Dioxetane compounds are enzymatic glow-type chemiluminescent substrates and are a class of ultra-sensitive substrates for alkaline phosphatase (Alkaline phosphatase, AP). In a suitable buffer solution, with catalytic hydrolysis effect of alkaline phosphatase, signals emitted by decomposition of dioxetane compounds can last for more than 20 hours, making the dioxetane compounds ideal chemiluminescent substances.

The present inventors have discovered that a chemiluminescent substrate solution with a ratio of the number of photons generated per second at the upper detection limit to that at the lower detection limit reaching 30,000 or more, even 50,000 or more, can be obtained from a chlorinated dioxetane compound having a spiroadamantane substituent when combined with a specific chemiluminescence enhancer such as a fluorescein or a carboxylic acid derivative thereof in the presence of a water-soluble polymeric quaternary ammonium salt cationic surfactant, which can be conveniently applied to the immunoassay of samples such as HCG where the concentration range of the analyte can span up to 10⁵-10⁶ orders of magnitude.

As referred to herein, "a dioxetane compound having a spiro-adamantane substituent" refers to a class of compounds based on the following structure:

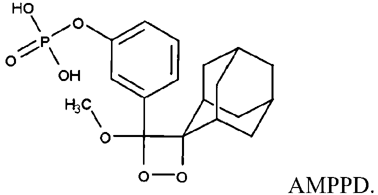

For example, the compounds include, but are not limited to, the following compounds:
AMPPD-(3-(2'-spiroadamatane)-4-methoxy-4-(3"-phosphoryloxy)-phenyl-1,2-dioxetane, CAS=122341-56-4),
CSPD-(3-(2'-(spiro-5-chloroadamantane))-4-methoxy-4-(3"-phosphoryloxy)-phenyl-1,2-dioxetane, CAS=142456-88-0),
ADP-STAR-(3-(2'-spiroadamatane)-4-methoxy-4-(3"-phosphoryloxy-4"-chloro)-phenyl-1,2-dioxetane, CAS=189942-84-5),
CDP-STA-(3-(2'-(spiro-5-chloroadamantane))-4-methoxy-4-(3"-phosphoryloxy-4"-chloro)-phenyl-1,2-dioxetane, CAS=160081-62-9),
TFE-AMPPD-(3-(2'-spiroadamatane)-4-trifluoroethoxy-4-(3"-phosphoryloxy)-phenyl-1,2-dioxetane).

As referred to herein, "a chlorinated dioxetane compound having a spiroadamantane substituent" refers to a compound containing at least one chlorine atom in the structure of AMPPD.

In some embodiments, the chlorinated dioxetane compound having a spiroadamantane substituent is one of ADP-STAR and CDP-STAR.

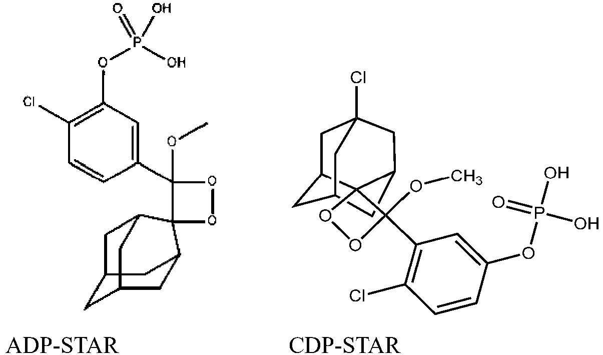

In some specific embodiments, in the chemiluminescent substrate solution, the chemiluminescent substrate may exist in the form of a salt of the aforementioned compound. According to some embodiments, the salt may be an alkali metal salt, such as a sodium salt.

These dioxetane compounds having chloro groups, especially ADP-STAR and CDP-STAR, when used in an alkaline-phosphatase-catalyzed system in combination with fluorescein or a carboxyl derivative thereof and a water-soluble polymeric quaternary ammonium salt, unexpectedly can provide sufficiently low sensitivity while still maintaining linearity in a relatively high luminescence value range.

The fluorescent agent and the surfactant can improve efficiency of chemiluminescence. The surfactant forms micelles in the solution to protect the chemiluminescent substrate, thereby reducing a quenching reaction of the chemiluminescent substrate in an aqueous solution. The fluorescent agent, as a photon acceptor, receives photon energy in the system through energy transfer effect and is excited to generate an optical signal, thereby enhancing luminescence efficiency.

In the chemiluminescent substrate solution of the present disclosure, the fluorescent agent is at least one of fluorescein and carboxyl-substituted fluorescein.

The carboxyl-substituted fluorescein includes a compound represented by general formula I:

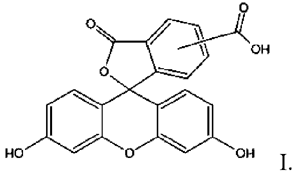

Specifically, the carboxyfluorescein represented by general formula I may be 5-carboxyfluorescein, 6-carboxyfluorescein, or 5(6)-carboxyfluorescein. wherein,
the 5-carboxyfluorescein is 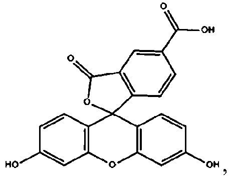
the 6-carboxyfluorescein is 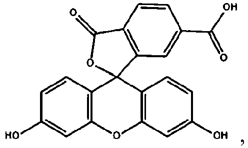
the 5(6)-carboxyfluorescein is a mixture in any ratio of the 5-carboxyfluorescein and the 6-carboxyfluorescein.

In the chemiluminescent substrate solution, the fluorescent agent is fluorescein. In other embodiments, the fluorescent agent may be any carboxyl-substituted fluorescein, particularly one of 5-carboxyfluorescein, 6-carboxyfluorescein, and 5(6)-carboxyfluorescein.

Fluorescein and a carboxyl derivative thereof can improve the chemiluminescence efficiency of a chemiluminescence system. In addition to effectively improving the chemiluminescence efficiency of the chemiluminescence system, the above carboxyl derivatives can also reduce the time required to reach a plateau phase and improve sensitivity.

In the chemiluminescent substrate solution, the surfactant is a water-soluble polymeric quaternary ammonium salt cationic surfactant. According to one embodiment, the water-soluble polymeric quaternary ammonium salt is selected from polyvinyl-containing quaternary ammonium salts. Such quaternary ammonium salts are, for example, polyvinylbenzyl-trihydrocarbylammonium halide. The three hydrocarbyl groups substituted on the N atom may be the same or different, and may be independently selected from, for example, methyl, ethyl, propyl, butyl, pentyl, benzyl, and the like. In specific embodiments, the water-soluble polymeric quaternary ammonium salt is selected from at least one of polyvinylbenzyl-trimethylammonium chloride, polyvinylbenzyl-benzyldimethylammonium chloride, and polyvinylbenzyltributylammonium chloride.

The water-soluble polymeric quaternary ammonium salt cationic surfactant can likewise form micelles in an aqueous solution; the chemiluminescent substrate is in the hydrophobic portion inside the micelles, while the fluorescent agent is in the hydrophilic portion outside the micelles, which more favourably facilitates energy transfer from the chemiluminescent substrate to the fluorescent agent, thereby enhancing the optical signal. Compared with small-molecule quaternary ammonium salts, since polymer quaternary ammonium salts have longer alkane chains, they can more effectively prevent the chemiluminescent substrate from being quenched in an aqueous solution. In addition, the polymer quaternary ammonium salt can also lower background signals and improve detection signals. By contrast, small-molecule quaternary ammonium salts under the same conditions have high background signal values and relatively low detection signals, and cannot achieve a wide linear detection range.

In one specific embodiment, the chemiluminescent substrate solution includes ADP-STAR or CDP-STAR as the chemiluminescent substrate, fluorescein, and a water-soluble polymeric quaternary ammonium salt.

In another specific embodiment, the chemiluminescent substrate solution includes ADP-STAR, fluorescein or carboxyl-substituted fluorescein as the fluorescent agent, and a water-soluble polymeric quaternary ammonium salt.

The above specific combinations of the chemiluminescent substrate, the fluorescent agent, and the surfactant provides a chemiluminescent substrate solution having a wide linear luminescence value range. The chemiluminescent substrate solution, when used in an alkaline phosphatase system, can achieve a linear detection range with a lower limit B1 of 3,000 photon counts per second or less and an upper limit B2 of 100,000,000 (100M) photon counts per second or more. As an example, the chemiluminescent substrate solution, when used in an alkaline phosphatase system, can achieve a linear detection range with a lower limit B1 of 1,000-3,000 photon counts per second and an upper limit B2 of 100,000,000 (100M) - 200,000,000 (200M) photon counts per second. As shown in the following examples, the chemiluminescent substrate solutions, when used in an alkaline phosphatase system, can achieve a linear detection range with a background luminescence value of 1,000-2,000 photon counts per second and an upper limit B2 of 120,000,000 (120M) - 200,000,000 (200M) photon counts per second. It should be understood that the ranges of the lower limit and the upper limit mentioned above are merely exemplary, and a lower limit of fewer photon counts per second or an upper limit of more photon counts per second may be obtained by, for example, adjusting the detection device or signal processing methods.

In some embodiments, the ratio of the number of photons generated per second at the upper detection limit B2 to that at the lower detection limit B1 of the chemiluminescent substrate solution is 30,000 or more, for example, 50,000 or more, even 60,000 or more. In some embodiments, the ratio of the number of photons generated per second at the upper detection limit B2 to that at the lower detection limit B1 of the chemiluminescent substrate solution is 1,000,000 or less, such as 800,000 or less, 600,000 or less, 500,000 or less, even 400,000 or less. As an example, the ratio of the number of photons generated per second at the upper detection limit B2 to that at the lower detection limit B1 of the chemiluminescent substrate solution is 30,000-1,000,000, 30,000-800,000, 30,000-500,000, 50,000-1,000,000, 50,000-800,000, 50,000-500,000, etc.

In some embodiments, the chemiluminescent substrate solution includes 50 to 500 mg/L of the chemiluminescent substrate. Preferably, the chemiluminescent substrate solution includes 150 to 250 mg/L of the chemiluminescent substrate, more preferably 200 mg/L of the chemiluminescent substrate. Exemplarily, the chemiluminescent substrate solution includes 150 mg/L, 160 mg/L, 170 mg/L, 180 mg/L, 190 mg/L, 200 mg/L, 210 mg/L, 220 mg/L, 230 mg/L, 240 mg/L, or 250 mg/L of the chemiluminescent substrate.

In some embodiments, the chemiluminescent substrate solution includes 30 to 500 mg/L of the fluorescent agent. Preferably, the chemiluminescent substrate solution includes 150 to 250 mg/L of the fluorescent agent, more preferably 200 mg/L of the fluorescent agent. Exemplarily, the chemiluminescent substrate solution includes 150 mg/L, 160 mg/L, 170 mg/L, 180 mg/L, 190 mg/L, 200 mg/L, 210 mg/L, 220 mg/L, 230 mg/L, 240 mg/L, or 250 mg/L of the fluorescent agent.

In some embodiments, the chemiluminescent substrate solution includes 1 to 10 g/L of the water-soluble polymeric quaternary ammonium salt. Preferably, the chemiluminescent substrate solution includes 3 to 7 g/L of the water-soluble polymeric quaternary ammonium salt, more preferably 5 g/L of the water-soluble polymeric quaternary ammonium salt. Exemplarily, the chemiluminescent substrate solution includes 3 g/L, 4 g/L, 5 g/L, 6 g/L, or 7 g/L of the water-soluble polymeric quaternary ammonium salt.

In one specific embodiment, the chemiluminescent substrate solution includes 50 to 500 mg/L, preferably 150 to 250 mg/L, of ADP-STAR or CDP-STAR, 30 to 500 mg/L, preferably 150 to 250 mg/L, of fluorescein, and 1 to 10 g/L, preferably 3 to 7 g/L, of the water-soluble polymeric quaternary ammonium salt.

In another specific embodiment, the chemiluminescent substrate solution includes 50 to 500 mg/L, preferably 150 to 250 mg/L, of ADP-STAR, 30 to 500 mg/L, preferably 150 to 250 mg/L, of fluorescein or carboxyl-substituted fluorescein, and 1 to 10 g/L, preferably 3 to 7 g/L, of the water-soluble polymeric quaternary ammonium salt.

The chemiluminescent substrate solution further contains additives such as a buffer and a preservative.

The type of the buffer is not particularly limited in the present disclosure, and any conventionally applicable buffer can be used in the chemiluminescent substrate solution of the present disclosure. In some embodiments, the buffer may be selected from a Tris buffer solution, an AMP buffer solution (AMP is 2-amino-2-methylpropanol), an AMPD buffer solution (AMPD is 2-amino-2-methyl-1,3-propanediol), a DEA buffer solution (DEA is diethanolamine), a CHES buffer solution (CHES is 2-(N-cyclohexylamino)ethanesulfonic acid), a Mopso buffer solution, an imidazole buffer solution, a phosphoric acid buffer solution, a carbonic acid buffer solution, a malic acid buffer solution, and a glycine buffer solution, but is not limited thereto. Exemplarily, the buffer may be selected from at least one of Tris-HCl, AMP-HCl, AMPD-HCl, DEA-HCl, CHES-HCl, boric acid-NaOH, and glycine-NaOH buffer systems. Preferably, the buffer is selected from an AMP-HCl buffer system. The AMP-HCl buffer system can exert a better buffering effect within a pH range of 9.0 to 10.0, and studies have shown that it is beneficial to the stability of chemiluminescent substrate molecules.

According to some embodiments, the pH of the buffer is 7.1 to 10.6, preferably about 9.0 to about 10.0, particularly preferably about 9.5.

The amount of the buffer is not particularly limited in the present disclosure, and can be determined according to the selected buffer system.

The type of the preservative is not particularly limited in the present disclosure, and preservatives conventionally used for detection reagents can all be used in the present disclosure. Examples include: sodium azide, Proclin series, potassium sorbate, sodium benzoate, BND, erythromycin, gentamicin, and the like, but are not limited thereto. Sodium azide and Proclin series are preferred.

The amount of the buffer is not particularly limited in the present disclosure, and can be determined according to the selected type of preservative.

In some embodiments, the chemiluminescent substrate solution may further include magnesium chloride. For example, 0.1 to 2 mM of magnesium chloride may be included.

According to one specific embodiment, the chemiluminescent substrate solution includes 50 to 500 mg/L, preferably 150 to 250 mg/L, of ADP-STAR or CDP-STAR; 30 to 500 mg/L, preferably 150 to 250 mg/L, of fluorescein; 1 to 10 g/L, preferably 3 to 7 g/L, of the water-soluble polymeric quaternary ammonium salt; an appropriate amount (such as 30 to 500 mM) of AMP-HCl buffer solution; an appropriate amount (such as 0.5 to 2 g/L) of sodium azide, with a pH of about 9.0 to about 10.0, such as about 9.5.

In another specific embodiment, the chemiluminescent substrate solution includes 50 to 500 mg/L, preferably 150 to 250 mg/L, of ADP-STAR; 30 to 500 mg/L, preferably 150 to 250 mg/L, of fluorescein or carboxyl-substituted fluorescein; 1 to 10 g/L, preferably 3 to 7 g/L, of the water-soluble polymeric quaternary ammonium salt; an appropriate amount (such as 30 to 500 mM) of AMP-HCl buffer solution; an appropriate amount (such as 0.5 to 2 g/L) of sodium azide, with a pH of about 9.0 to about 10.0, such as about 9.5.

According to some embodiments, the chemiluminescent substrate solution of the present disclosure has a low background luminescence value, a high luminescence efficiency, and a wide linear detection range, which is suitable for an alkaline-phosphatase-catalyzed luminescence system, especially applicable to chemiluminescent immunoassay.

According to some specific embodiments, the chemiluminescent substrate solution provided by the present disclosure reacts with an alkaline phosphatase with a concentration range of 0.03-400 ng/mL, and the luminescence values exhibit good linearity (R² ≥ 0.99). As shown in the examples, background values of the chemiluminescent substrate solution may be as low as 3,000 photon counts per second or less, making it possible to detect an alkaline phosphatase with an ultra-low concentration reaching 10-19 mol/L, thereby improving the detection sensitivity. Additionally, detection values of the chemiluminescent substrate solution may be as high as 100M photon counts per second or more, such as 100M-200M photon counts per second and 120M-200M photon counts per second, which exhibit a significantly wider linearity. The above chemiluminescent substrate solution can satisfy detection of a sample with high analyte concentration, without additionally diluting the sample.

The chemiluminescent substrate solution of the present disclosure is particularly suitable for detecting human chorionic gonadotropin HCG in a sample to be tested.

HCG is a glycoprotein mainly secreted by placental trophoblast cells, and is present at a very low level in non-pregnant individuals, usually less than 3 mIU/ml, whereas during pregnancy, the HCG concentration can be as high as about 2,000,000 mIU/ml. Clinically, HCG test results are used to assist in determining whether pregnancy exists. Meanwhile, HCG detection during pregnancy is also of great significance. As described above, the chemiluminescent substrate solution of the present disclosure has wide-linear luminescence characteristics and is suitable for a sample in which the concentration of an analyte such as HCG exhibits a large variation range.

According to some embodiments, the concentration range of the HCG in the sample is 1 to 200,000 mIU/mL.

In some embodiments, the detection of HCG is performed by a double-antibody sandwich method. According to specific embodiments, superparamagnetic particles coated with an anti-HCG antibody are used as the capture reagent in the detection, and an alkaline-phosphatase-labeled anti-HCG antibody is used as the label. In some embodiments, the anti-HCG antibody is a monoclonal antibody.

The chemiluminescent substrate solution of the present disclosure, in combination with the double-antibody sandwich method, provides high sensitivity and a wide linear detection range for HCG detection.

In the present disclosure, when the chemiluminescent substrate solution is used in an immunoassay of HCG, specific forms and amounts of corresponding immunoassay reagents, such as the HCG antibody, the specific capture reagent, and the alkaline phosphatase label, are not particularly limited, and any suitable reagents may be used.

As used herein, "sample", unless otherwise specified, refers to a biological sample, which may be derived from a mammal, preferably a blood sample derived from a human, and more preferably a serum sample.

In addition to HCG, the chemiluminescent substrate solution of the present disclosure is also suitable for detecting other samples with large concentration ranges of the analyte, such as HBsAg and TSH, not limited to HCG.

In some embodiments, the above chemiluminescent substrate solution is used to perform a chemiluminescence detection method in the chemiluminescence analyser of the embodiments of the present disclosure. The chemiluminescence detection method includes steps of:
mixing a sample to be tested with a detection reagent including a capture reagent and an alkaline phosphatase label that are capable of binding to an analyte to obtain an alkaline-phosphatase-labeled immune complex;
mixing the alkaline phosphatase-labeled immune complex with the chemiluminescent substrate solution according to any of the above embodiments, thereby obtaining a mixed solution;
measuring an optical signal of the mixed solution, and obtaining an analysis result of the sample to be tested based on the optical signal.

Some embodiments of the photometer of the chemiluminescence analyser of the present disclosure are described below, but the present disclosure is not limited thereto.

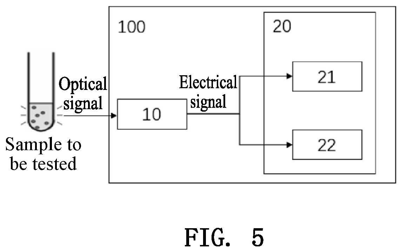

In some embodiments, as shown in FIG. 5, the photometer includes a receiving component 10 and a processing component 20 that are electrically connected to each other.

The receiving component 10 is configured to receive the optical signal generated in the chemiluminescent reaction and convert the optical signal into a corresponding electrical signal. The receiving component 10 may be configured, for example, as a photomultiplier tube that converts a weak optical signal into an electrical signal.

The processing component 20 is configured to be electrically connected to the receiving component 10 so as to receive the electrical signal from the receiving component 10, and to process the received electrical signal so as to obtain the number of photons. The processing component 20 includes a first photon counting module 21 and a second photon counting module 22, wherein the first photon counting module 21 is configured to process the electrical signal by using a first photon counting method to obtain a first photon counting result, and the second photon counting module 22 is configured to process the electrical signal by using a second photon counting method different from the first photon counting method to obtain a second photon counting result. The processing component 20 is further configured to output a final photon counting result of the sample to be tested based on the first photon counting result and the second photon counting result and use the final photon counting result as the photon count outputted by the photometer. The final photon counting result, i.e., the photon count outputted by the photometer, can then be used to calculate the content of the analyte in the sample to be tested.

In the embodiments of the present disclosure, two photon counting modules are used to perform photon counting simultaneously, wherein the first photon counting module obtains the first photon counting result by using a first counting method for a weak light range, and the second photon counting module obtains the second photon counting result by using a second counting method for a strong light range, and finally the first photon counting result and the second photon counting result are integrated and a final counting result is outputted. Thus, the linear detection range of the photometer can be greatly expanded. In particular, the linear detection range of the photometer can be expanded at least to [2000, 100 million (10⁸)] photon counts per second, or even to [2000, 200 million (10⁸)] photon counts per second.

In the embodiments of the present disclosure, the weak light range may be understood as an optical signal whose photons per second is not higher than a predetermined threshold, and the strong light range may be understood as an optical signal whose photons per second is higher than the predetermined threshold. For example, the predetermined threshold may be selected from any value from 20 million to 30 million photon counts per second, for example may be 20 million or 30 million photon counts per second. For example, the first counting method is a photon counting method for an optical signal whose photon counts per second is not higher than 30 million photon counts per second, and the second counting method is a photon counting method for an optical signal whose photon counts per second is higher than 30 million photon counts per second.

In some embodiments, the first counting method may be a pulse recognition method for calculating photon counts by recognizing electrical pulses caused by photons entering the receiving component, and the second counting method does not calculate photon counts by recognizing electrical pulses caused by photons entering the receiving component, but estimates the number of photons by processing the electrical signal into a parameter capable of representing the number of photons in the optical signal (i.e., the number of electrical pulses caused by photons entering the receiving component).

Here, those skilled in the art can understand that, in the examples of the present disclosure, the pulse recognition method may be understood as a method for calculating photon counts by recognizing pulses in an electrical signal, that is, when a peak value of a pulse recognized from the electrical signal is greater than a threshold, it is considered that a photon is recognized.

In some embodiments, the first photon counting module 21 may be configured to detect the number of pulses of the electrical signal by using the pulse recognition method to obtain the first photon counting result, and the second photon counting module 22 may be configured to process the electrical signal to obtain a parameter characterizing the number of photons in the optical signal, and obtain, for example calculate, the second photon counting result according to the parameter characterizing the number of photons and a preset calibration function, wherein the preset calibration function represents a mapping relationship between the parameter characterizing the number of photons in the optical signal and the photon counting result.

In some embodiments, after obtaining the first photon counting result and the second photon counting result, the processing component 20 may be further configured to:
when the first photon counting result is lower than a first threshold, use the first photon counting result as the final photon counting result, i.e., the photon count outputted by the photometer; and
when the first photon counting result is not lower than the first threshold, use the second photon counting result as the final photon counting result, i.e., the photon count outputted by the photometer.

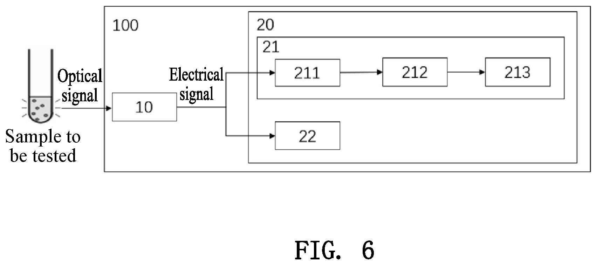

In some embodiments, as shown in FIG. 6, the first photon counting module 21 may include an electrical level discrimination circuit 211, a shaping and frequency division circuit 212, and a first counting circuit 213 that are electrically connected to each other. The electrical level discrimination circuit 211 is configured to convert the electrical signal into a square wave signal, the shaping and frequency division circuit 212 is configured to perform frequency division and shaping on the square wave signal, and the first counting circuit 213 is configured to perform counting, i.e., recognize the number of pulses on the signal outputted by the shaping and frequency division circuit 212, to obtain the first photon counting result. The first photon counting module 21 according to this example can achieve a linear detection range of 2,000 to 30 million photon counts per second.

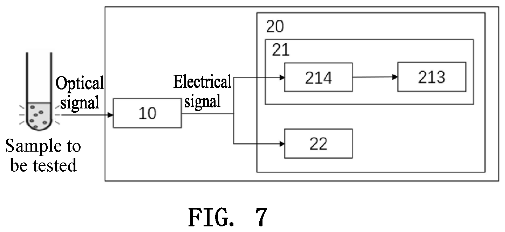

In some other alternative examples, as shown in FIG. 7, the first photon counting module 21 may include at least one first AD conversion circuit 214 and a first counting circuit 213 that are electrically connected to each other. The first AD conversion circuit 214 is configured to acquire the electrical signal at a sampling frequency greater than 1 GHz/s and convert the acquired electrical signal into a digital signal for output to the first counting circuit 213, and the first counting circuit 213 is configured to analyse the received digital signal to recognize the number of pulses, thereby obtaining the first photon counting result. Thus, by high-speed sampling at a sampling frequency of 1 GHz/s, the first counting circuit 213 can more accurately recognize the number of pulses in the electrical signal, thereby more accurately obtaining the first photon counting result. Compared with the example shown in FIG. 6, the first photon counting module 21 according to this example can achieve a linear detection range of 2,000 to 80 million photon counts per second.

In some variations of the example shown in FIG. 7, the first AD conversion circuit may be configured to acquire the electrical signal at a sampling frequency not less than 2 GHz/s. Thus, the linear detection range of the first photon counting module 21 can be further broadened.

In some variations of the example shown in FIG. 7, the first photon counting module 21 may include at least two, in particular at least four, first AD conversion circuits 214, a sum of sampling frequencies of the first AD conversion circuits is not less than 1 GHz/s, and the first AD conversion circuits acquire the electrical signal by asynchronous acquisition at preset time intervals. By using a plurality of first AD conversion circuits to achieve a sampling frequency not less than 1 GHz/s, performance requirements for a single AD conversion circuit can be reduced, thereby reducing costs.

Preferably, these first AD conversion circuits 214 have a same sampling frequency, and the first AD conversion circuits acquire the electrical signal by asynchronous acquisition at equal time intervals.

Correspondingly, the first counting circuit 213 may be configured to rearrange and combine data acquired by the plurality of first AD conversion circuits 214 described above, perform filtering processing, and then perform pulse recognition on the filtered signal to obtain the first photon counting result.

Further, the first counting circuit 213 may further be configured to correct the first photon counting result by a Poisson distribution compensation algorithm. Thus, a more accurate photon counting result can be obtained.

In some embodiments, the first counting circuit 213 may include an FPGA chip and peripheral circuits thereof.

In some embodiments, the second photon counting module 22 may be configured to process the electrical signal, for example by integration processing, to obtain a direct current component signal, and the parameter characterizing the number of photons in the optical signal includes a parameter related to the direct current component signal, for example the direct current component signal itself per unit time. The second photon counting module 22 is further configured to obtain the second photon counting result according to the parameter related to the direct current component signal and the preset calibration function.

Alternatively or additionally, the second photon counting module 22 is configured to integrate the electrical signal within a predetermined time period to obtain an integration result, for example a direct current component signal, and the parameter characterizing the number of photons in the optical signal includes a parameter related to the integration result, for example the integration result per unit time. The second photon counting module 22 is further configured to obtain the second photon counting result according to the parameter related to the integration result and the preset calibration function.

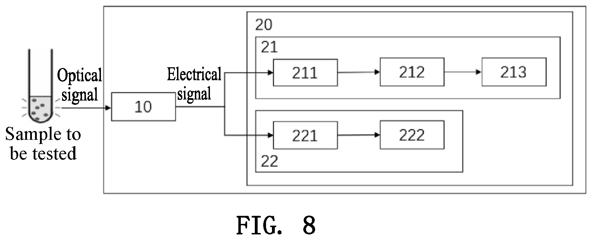

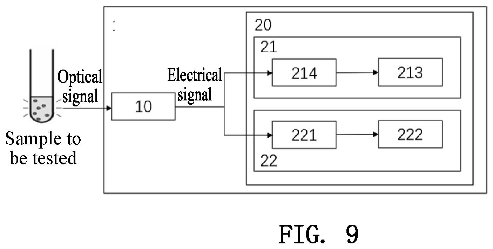

In a specific example, as shown in FIG. 8 and FIG. 9, the second photon counting module 22 includes an integration circuit 221 and a second counting circuit 222 that are electrically connected to each other. The integration circuit 221 is configured to integrate the electrical signal within a predetermined time period to obtain a direct current component signal, and the parameter characterizing the number of photons in the optical signal includes a parameter related to the direct current component signal. The second counting circuit 222 is configured to obtain the second photon counting result according to the parameter related to the direct current component signal and the preset calibration function.

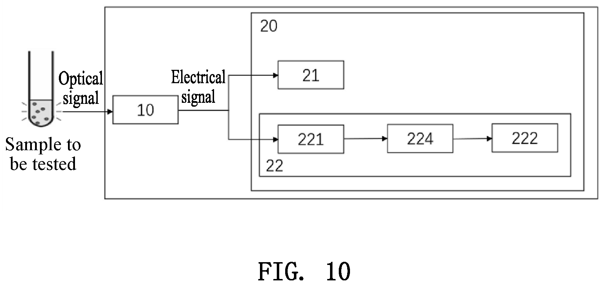

Further, as shown in FIG. 10, an AD conversion circuit 224 is further provided between the integration circuit 221 and the second counting circuit 222, and the AD conversion circuit 224 is configured to convert the direct current component signal outputted by the integration circuit 221 into a digital signal. The sampling frequency of the AD conversion circuit 224 may be set to less than 1 MHz/s, for example several hundred kHz/s.

In some embodiments, the second counting circuit 222 may include an FPGA chip and peripheral circuits thereof.

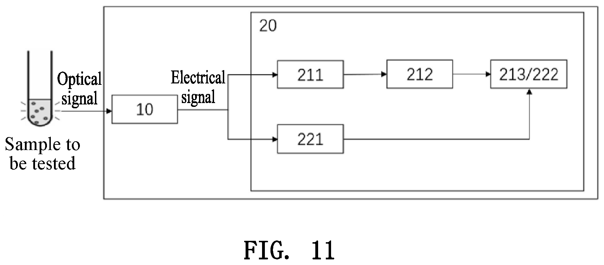

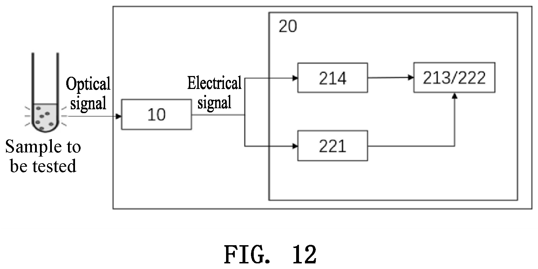

Preferably, as shown in FIG. 11 and FIG. 12, the first counting circuit 213 and the second counting circuit 222 are the same counting circuit, and the same counting circuit includes, for example, an FPGA chip. That is, the counting functions of the first photon counting module 21 and the second photon counting module 22 are integrated in the same FPGA chip; that is, the FPGA chip is configured to, on the one hand, perform counting on the signal outputted by the shaping and frequency dividing circuit 212 to obtain the first photon counting result (FIG. 11) or analyse the digital signal acquired by the first AD conversion circuit 214 to obtain the first photon counting result (FIG. 12), and, on the other hand, obtain the second photon counting result according to the parameter characterizing the number of photons in the optical signal and the preset calibration function.

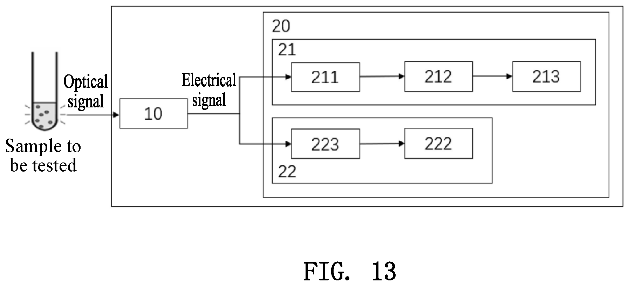

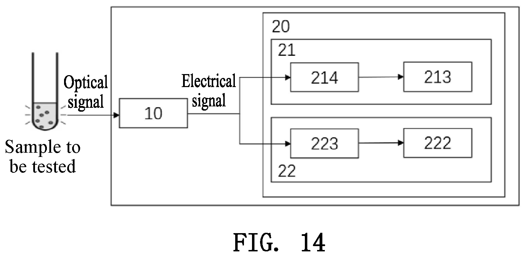

In another alternative example, as shown in FIG. 13 and FIG. 14, the second photon counting module includes a second AD conversion circuit 223 and a second counting circuit 222 that are electrically connected to each other. The second AD conversion circuit is configured to convert the electrical signal outputted by the receiving component 10 into a digital signal, and the second counting circuit is configured to process the digital signal to obtain the parameter characterizing the number of photons in the optical signal, and obtain the second photon counting result according to the parameter characterizing the number of photons in the optical signal and the preset calibration function.

Here, the second AD conversion circuit 223 having a sampling frequency less than 10 MHz/s may be used. In some embodiments, the sampling frequency of the second AD conversion circuit 223 may be within a range of 1 MHz/s to 10 MHz/s, for example 1, 2, or 3 MHz/s.

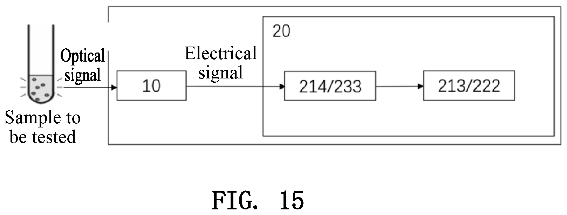

Preferably, as shown in FIG. 15, the first counting circuit 213 and the second counting circuit 222 are the same counting circuit, and the same counting circuit includes, for example, an FPGA chip. That is, the counting functions of the first photon counting module 21 and the second photon counting module 22 are integrated in the same chip.

Also preferably, as shown in FIG. 15, the first AD conversion circuit 214 is used as the second AD conversion circuit 223, that is, the first photon counting module 21 and the second photon counting module 22 use the same AD conversion circuit.

Further, the second counting circuit 222 may be configured to process the digital signal to obtain a direct current component signal, and the parameter characterizing the number of photons in the optical signal includes a parameter related to the direct current component signal.

For example, the second counting circuit 222 may be configured to perform Fourier transform on the digital signal to obtain the direct current component signal.

Alternatively or additionally, the second counting circuit 222 may be configured to integrate or sum the digital signal within a predetermined time period to obtain a parameter related to the integration result or the summation result and use said parameter as the parameter characterizing the number of photons in the optical signal.

For more examples of the photometer of the present disclosure, reference may be made to Chinese prior application CN 202211510485.X of the present applicant, the contents of which are incorporated into the present disclosure by reference.

A third embodiment of the present disclosure provides another chemiluminescence analyser, including: a sample preparation device, configured to cause a blood sample to be tested to be mixed with reaction reagents in a reaction vessel, so that an analyte in the blood sample to be tested reacts with the reaction reagents to obtain a sample solution to be tested; a substrate providing device, configured to supply a luminescent substrate into the reaction vessel containing the sample solution to be tested, so that the sample solution to be tested undergoes a chemiluminescent reaction with the luminescent substrate; a photometric device, at least comprising a photometer configured to detect a luminescence signal generated in the chemiluminescent reaction; a data processing device, configured to obtain a test result of the analyte in the blood sample to be tested based on the luminescence signal and a preset calibration model.

Herein, the photometer comprises a receiving component configured to receive the optical signal generated in the chemiluminescent reaction and convert the optical signal into a corresponding electrical signal, and a processing component configured to be electrically connected to the receiving component and receive the electrical signal from the receiving component, wherein the processing component comprises a first photon counting module configured to detect a number of pulses of the electrical signal by using a pulse recognition method to obtain a first photon counting result, and a second photon counting module configured to process the electrical signal to obtain a parameter characterizing a number of photons in the optical signal, and obtain a second photon counting result based on the parameter characterizing the number of photons in the optical signal and a preset calibration function, wherein the preset calibration function represents a mapping relationship between the parameter characterizing the number of photons in the optical signal and a photon counting result, and the processing component is further configured to obtain a photon count outputted by the photometer based on the first photon counting result and the second photon counting result, and output the photon count; and a second linear detection range of the luminescent substrate is [B1, B2] photon counts per second, the second linear detection range represents a range of the intensity of the luminescence signal generated in the chemiluminescent reaction, and within the second linear detection range, a number of the labels participating in the chemiluminescent reaction is in a linear relationship with the intensity of the luminescence signal generated in the chemiluminescent reaction, wherein a ratio of B2 to B1 is greater than or equal to 30000 or B1 is less than or equal to 3000 and B2 is greater than or equal to 10⁸.

For more embodiments and advantages of the chemiluminescence analyser according to the third embodiment of the present disclosure, reference may be made to the above descriptions of the chemiluminescence analysers according to the first embodiment and the second embodiment of the present disclosure.

A fourth embodiment of the present disclosure provides yet another chemiluminescence analyser, including: a sample preparation device, configured to cause a blood sample to be tested to be mixed with reaction reagents in a reaction vessel, so that an analyte in the blood sample to be tested reacts with the reaction reagents to obtain a sample solution to be tested; a substrate providing device, configured to supply a luminescent substrate into the reaction vessel containing the sample solution to be tested, so that the sample solution to be tested undergoes a chemiluminescent reaction with the luminescent substrate; a photometric device, at least comprising a photometer configured to detect a luminescence signal generated in the chemiluminescent reaction; a data processing device, configured to obtain a test result of the analyte in the blood sample to be tested based on the luminescence signal and a preset calibration model.

Herein, a first linear detection range of the photometer is [A1, A2] photon counts per second, the first linear detection range represents a range of photon counts outputted by the photometer, and within the first linear detection range, an intensity of the luminescence signal generated in the chemiluminescent reaction is in a linear relationship with photon counts outputted by the photometer, wherein a ratio of A2 to A1 is greater than or equal to 30000 or A1 is less than or equal to 2000 and A2 is greater than or equal to 10⁸, and the luminescent substrate comprises a chemiluminescent substrate and a chemiluminescence enhancer, the chemiluminescence enhancer comprises a fluorescent agent and a surfactant, and the fluorescent agent comprises one or more carboxyfluoresceins.

For more embodiments and advantages of the chemiluminescence analyser according to the fourth embodiment of the present disclosure, reference may be made to the above descriptions of the chemiluminescence analysers according to the first embodiment and the second embodiment of the present disclosure.

The features or combinations of features mentioned above in the description, drawings, and claims may be used in any mutual combination or alone, as long as they are meaningful within the scope of the present disclosure and do not contradict each other.

Various embodiments and advantages of the chemiluminescent substrate of the present disclosure are illustrated below by specific examples, but the scope of the present disclosure is not limited thereto.

Example 1: linearity performance using different chemiluminescent substrates formulation of chemiluminescent substrate solution:
Chemiluminescent substrate: 200 mg
5(6)-carboxyfluorescein: 200 mg
Poly(vinylbenzyltrimethylammonium chloride): 5 g
AMP-HCl: 50 mM
magnesium chloride: 200 mg
sodium azide: 1 g
water: added to a total volume of 1 L

According to the above formulation, wherein the chemiluminescent substrates are shown in Table 1 below, a series of chemiluminescent substrate solutions 1-1 to 1-4 were prepared.

**Table 1**

| chemiluminescent substrate solution | chemiluminescent substrate |
|---|---|
| 1-1 | AMPPD |
| 1-2 | CSPD |
| 1-3 | ADP-STAR |
| 1-4 | CDP-STAR |

The above chemiluminescent substrate solutions 1-1 to 1-4 were tested on a Mindray CL-6000i fully automatic chemiluminescence immunoassay analyser. Alkaline phosphatase (AP enzyme) solutions at different concentration gradients were respectively mixed with each chemiluminescent substrate solution, and after incubation for a period of time, optical signals were collected, thereby respectively obtaining background signal values (i.e., the alkaline phosphatase concentration was 0) and luminescence signal values under a light collection time of 2 min; the concentrations of alkaline phosphatase in the system and the detection signal values are shown in Table 2 below.

**Table 2**

| chemiluminescent substrate solution | 1-1 | 1-2 | 1-3 | 1-4 |
|---|---|---|---|---|
| chemiluminescent substrate | AMPPD | CSPD | ADP-STAR | CDP-STAR |
| concentration of AP (ng/mL) | RLU (photon counts per second) | | | |
| 0 | 4,736 | 904 | 1,681 | 807 |
| 0.03 | 21,318 | 10,592 | 21,252 | 23,163 |
| 0.5 | 268,768 | 147,952 | 314,652 | 339,886 |
| 5 | 2,490,726 | 1,536,422 | 3,020,790 | 3,358,141 |
| 50 | 22,934,439 | 17,851,702 | 23,898,506 | 25,088,083 |
| 100 | 40,045,569 | 34,585,418 | 43,174,174 | 48,425,597 |
| 200 | 65,992,786 | 60,178,500 | 86,677,025 | 86,423,603 |
| 400 | 98,451,533 | 100,426,348 | 167,526,515 | 149,275,704 |

The optical signals (photon counts per second) measured for the chemiluminescent substrate solutions 1-1 to 1-4 were respectively plotted against the concentrations of alkaline phosphatase in the system, and linear fitting was performed, as shown in FIG. 1A to FIG. 1D.

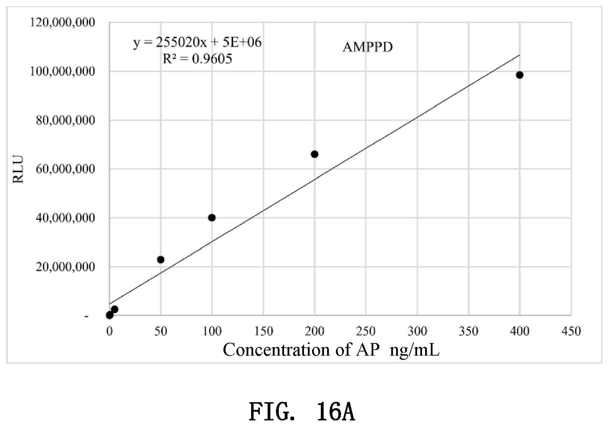

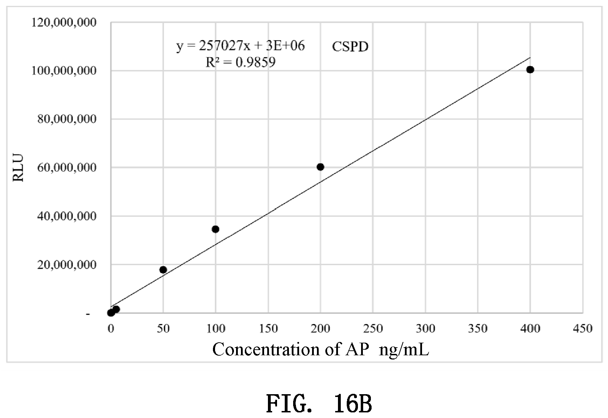

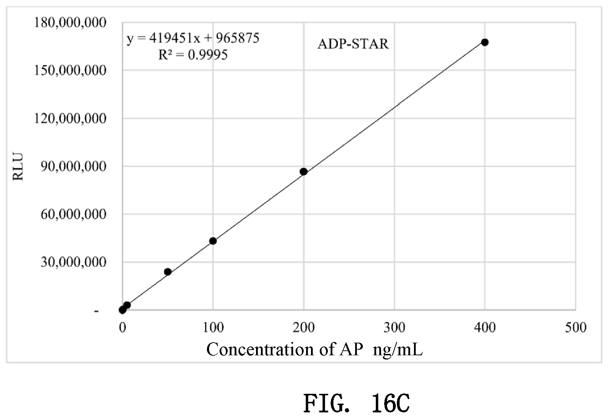

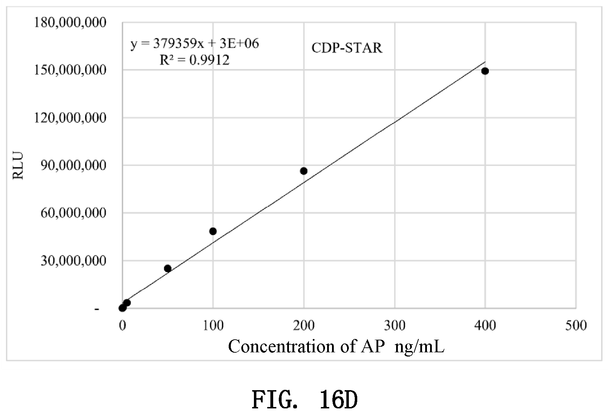

According to the data in Table 2 and FIG. 16A to FIG. 16D, the chemiluminescent substrate solutions 1-2, 1-3, and 1-4 have low background signals, while the chemiluminescent substrate solutions 1-3 and 1-4 also have the characteristics of high luminescence efficiency and good linearity (R² ≥ 0.99) within the detected luminescence range, wherein ADP-STAR performs best.

Example 2: linearity performance using ADP-STAR and different fluorescent agents
formulation of chemiluminescent substrate solution:
ADP-STAR: 200 mg
fluorescent agent: 200 mg
Poly(vinylbenzyltrimethylammonium chloride): 5 g
AMP-HCl: 50 mM
magnesium chloride: 200 mg
sodium azide: 1 g
water: added to a total volume of 1 L

According to the above formulation, the fluorescent agents are shown in Table 3 below, a series of chemiluminescent substrate solutions 2-1 to 2-4 were prepared.

**Table 3**

| chemiluminescent substrate solution | fluorescent agent |
|---|---|
| 2-1 | fluorescein |
| 2-2 | 5-carboxyfluorescein |
| 2-3 | 6-carboxyfluorescein |
| 2-4 | 5(6)-carboxyfluorescein |

The above chemiluminescent substrate solutions 2-1 to 2-4 were tested on a Mindray CL-6000i fully automatic chemiluminescence immunoassay analyser. Alkaline phosphatase solutions with different concentration gradients were respectively mixed with each chemiluminescent substrate solution, and after incubation for a period of time, optical signals were collected, thereby respectively obtaining background signal values (i.e., the alkaline phosphatase concentration was 0) and luminescence signal values under a light collection time of 2 min; the concentrations of alkaline phosphatase in the system and the detection signal values are shown in Table 4 below.

**Table 4**

| chemiluminescent substrate solution | 2-1 | 2-2 | 2-3 | 2-4 |
|---|---|---|---|---|
| fluorescent agent | fluorescein | 5-Carboxyfluorescein | 6-carboxyfluorescein | 5(6)-carboxyfluorescein |
| concentration of AP (ng/mL) | RLU (photon counts per second) | | | |
| 0 | 1,989 | 1,405 | 1,677 | 1,681 |
| 0.03 | 17,183 | 23,458 | 24,495 | 21,252 |
| 0.5 | 262,208 | 351,701 | 361,440 | 314,652 |
| 5 | 2,793,415 | 3,432,637 | 3,480,053 | 3,020,790 |
| 50 | 20,799,241 | 26,656,954 | 27,652,849 | 23,898,506 |
| 100 | 38,943,689 | 49,160,767 | 49,797,710 | 43,174,174 |
| 200 | 75,942,665 | 100,407,855 | 99,525,846 | 86,677,025 |
| 400 | 131,181,845 | 184,916,513 | 190,047,810 | 167,526,515 |

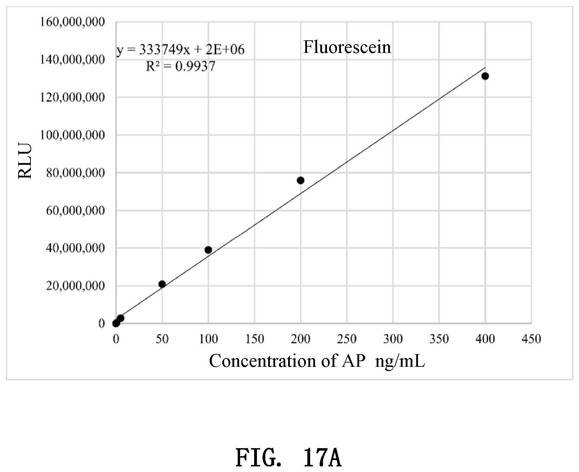

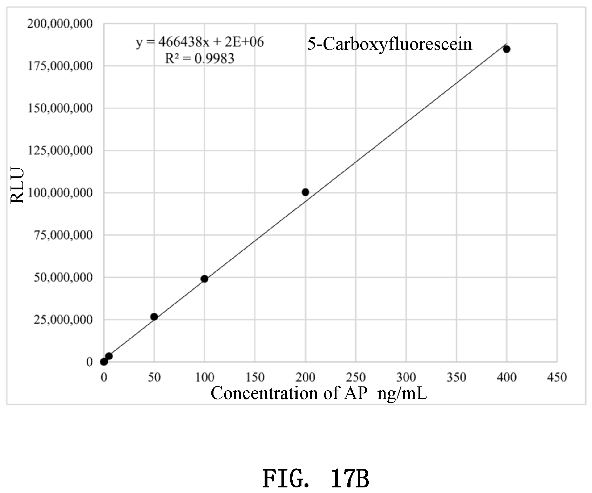

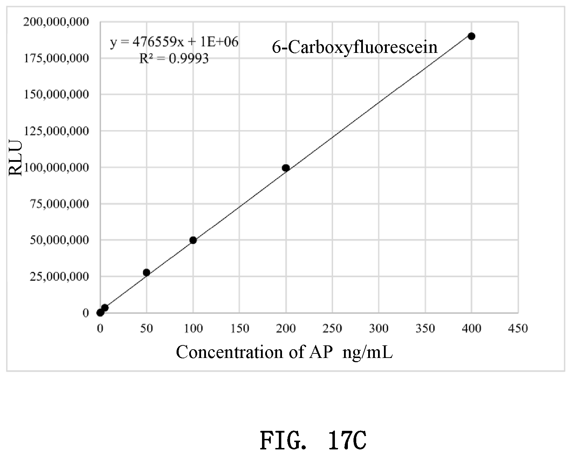

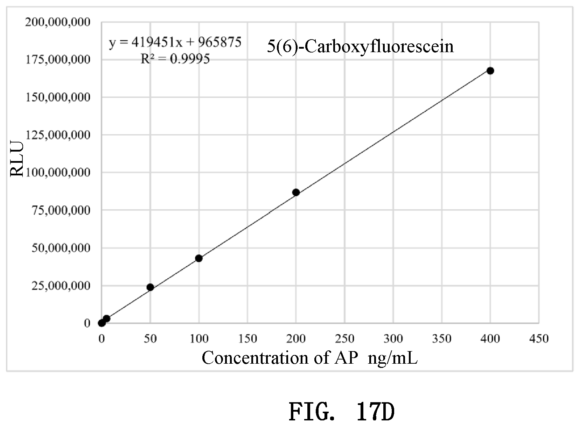

The optical signals (photon counts per second) measured for the chemiluminescent substrate solutions 2-1 to 2-4 were respectively plotted against the concentrations of alkaline phosphatase in the system, and linear fitting was performed, as shown in FIG. 17A to FIG. 17D.

According to the data in Table 4 and FIG. 17A to FIG. 17D, the chemiluminescent substrate solutions 2-1 to 2-4 all exhibit the characteristics of low background signals and high luminescence efficiency, and all have good linearity (R² ≥ 0.99) within the detected luminescence range.

Example 3: linearity performance using CDP-STAR and different fluorescent agents

A chemiluminescent substrate solution was prepared according to the following formulation:
CDP-STAR: 200 mg
fluorescent agent: 200 mg
Poly(vinylbenzyltrimethylammonium chloride): 5 g
AMP-HCl: 50 mM
magnesium chloride: 200 mg
sodium azide: 1 g
water: added to a total volume of 1 L

According to the above formulation, the fluorescent agents are shown in Table 5 below, a series of chemiluminescent substrate solutions 3-1 to 3-4 were prepared.

**Table 5**

| chemiluminescent substrate solution | fluorescent agent |
|---|---|
| 3-1 | fluorescein |
| 3-2 | 5-carboxyfluorescein |
| 3-3 | 6-carboxyfluorescein |
| 3-4 | 5(6)-carboxyfluorescein |

The above chemiluminescent substrate solutions 3-1 to 3-4 were tested on a Mindray CL-6000i fully automatic chemiluminescence immunoassay analyser. Alkaline phosphatase solutions with different concentration gradients were respectively mixed with each chemiluminescent substrate solution, and after incubation for a period of time, optical signals were collected, thereby respectively obtaining background signal values (i.e., the alkaline phosphatase concentration was 0) and luminescence signal values under a light collection time of 2 min; the concentrations of alkaline phosphatase in the system and the detection signal values are shown in Table 6 below.

**Table 6**

| chemiluminescent substrate solution | 3-1 | 3-2 | 3-3 | 3-4 |
|---|---|---|---|---|
| fluorescent agent | fluorescein | 5-Carboxyfluorescein | 6-carboxyfluorescein | 5(6)-carboxyfluorescein |
| concentration of AP (ng/mL) | RLU (photon counts per second) | | | |
| 0 | 1,389 | 798 | 1,013 | 807 |
| 0.03 | 18,728 | 25,567 | 26,964 | 23,163 |
| 0.5 | 283,236 | 379,907 | 390,426 | 339,886 |
| 5 | 3,105,373 | 3,815,982 | 3,868,692 | 3,358,141 |
| 50 | 21,834,549 | 27,983,836 | 29,029,303 | 25,088,083 |
| 100 | 43,680,543 | 54,140,360 | 55,854,776 | 48,425,597 |
| 200 | 75,720,627 | 97,292,611 | 101,103,837 | 86,423,603 |
| 400 | 131,890,525 | 176,771,186 | 179,343,466 | 149,275,704 |

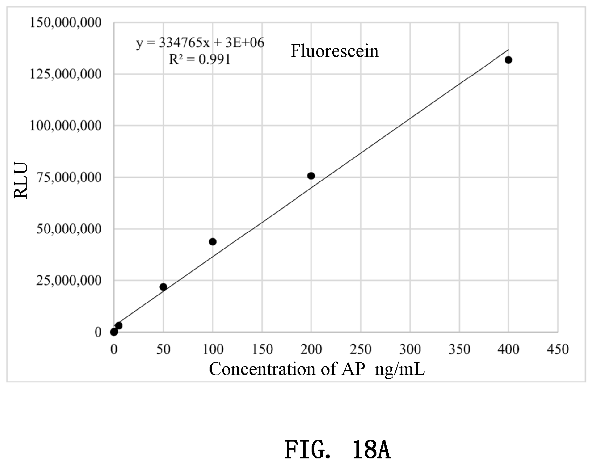

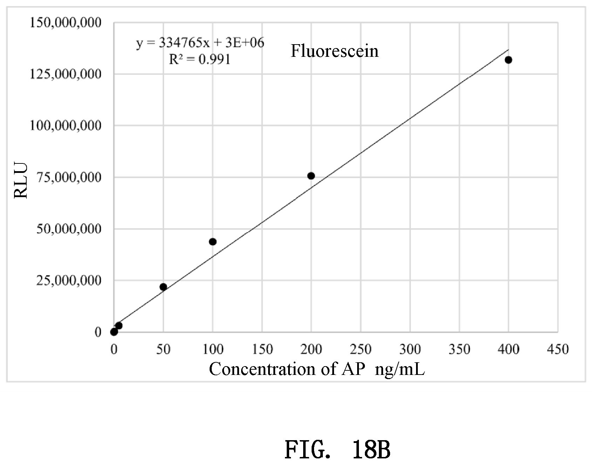

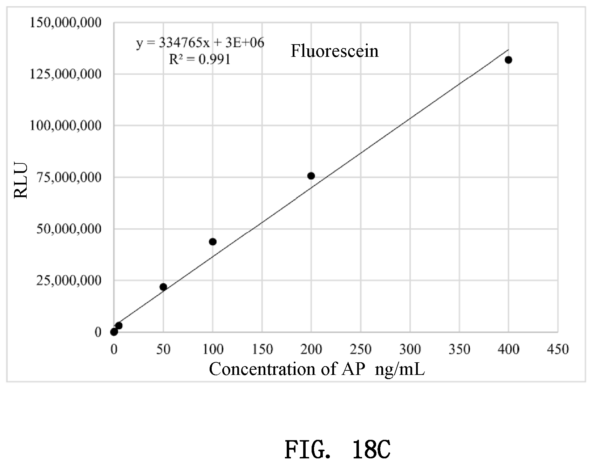

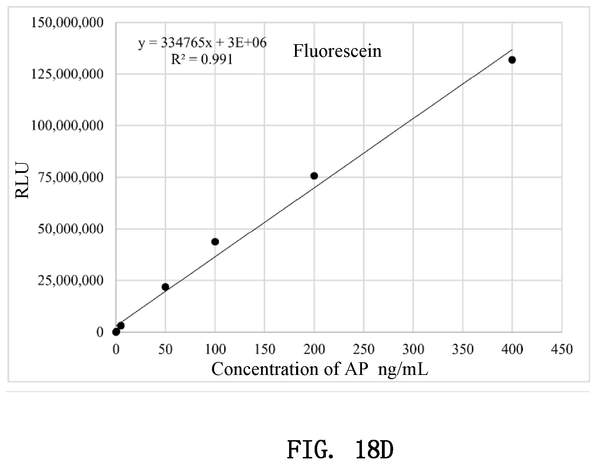

The optical signals (photon counts per second) measured for the chemiluminescent substrate solutions 3-1 to 3-4 were respectively plotted against the concentrations of alkaline phosphatase in the system, and linear fitting was performed, as shown in FIG. 18A to FIG. 18D.

According to the data in Table 6 and FIG. 18A to FIG. 18D, the chemiluminescent substrate solutions 3-1 to 3-4 all exhibit the characteristics of low background signals and high luminescence efficiency, and have good linearity (R² ≥ 0.99) within the detected luminescence range. Example 4: linearity performance using ADP-STAR and different quaternary ammonium salt cationic surfactants
formulation of chemiluminescent substrate solution:
ADP-STAR: 200 mg
fluorescein: 200 mg
quaternary ammonium salt surfactant: 5 g
AMP-HCl: 50 mM
magnesium chloride: 200 mg
sodium azide: 1 g
water: added to a total volume of 1 L

According to the above formulation, wherein the quaternary ammonium salt cationic surfactants are shown in Table 7 below, a series of chemiluminescent substrate solutions 4-1 to 4-4 were prepared.

**Table 7**

| chemiluminescent substrate solution | quaternary ammonium salt cationic surfactant |
|---|---|
| 4-1 | Poly(vinylbenzyltrimethylammonium chloride) (TMQ) |
| 4-2 | poly(vinylbenzyltributylammonium chloride) (TBQ) |
| 4-3 | poly(vinylbenzyl(benzyldimethyl)ammonium chloride) (BDMQ) |
| 4-4 | cetyltrimethylammonium bromide (CTAB) |

The above chemiluminescent substrate solutions **4-1 to 4-4** were tested on a Mindray CL-6000i fully automatic chemiluminescence immunoassay analyser. Alkaline phosphatase solutions with different concentration gradients were respectively mixed with each chemiluminescent substrate solution, and after incubation for a period of time, optical signals were collected, thereby respectively obtaining background signal values (i.e., the alkaline phosphatase concentration was 0) and luminescence signal values under a light collection time of 2 min; the concentrations of alkaline phosphatase in the system and the detection signal values are shown in Table 8 below.

**Table 8**

| chemiluminescent substrate solution | 4-1 | 4-2 | 4-3 | 4-4 |
|---|---|---|---|---|
| surfactant | TMQ | TBQ | BDMQ | CTAB |
| concentration of AP (ng/mL) | RLU | | | |
| 0 | 1,989 | 1,855 | 1,907 | 5,896 |
| 0.03 | 17,183 | 24,636 | 20,380 | 7,380 |
| 0.5 | 262,208 | 303,083 | 276,118 | 27,858 |
| 5 | 2,793,415 | 2,826,997 | 2,559,818 | 224,219 |
| 50 | 20,799,241 | 22,560,126 | 23,037,041 | 2,176,864 |
| 100 | 38,943,689 | 39,291,832 | 37,954,693 | 4,660,162 |
| 200 | 75,942,665 | 81,990,581 | 66,690,051 | 9,380,900 |
| 400 | 131,181,845 | 150,218,696 | 136,498,234 | 13,793,862 |

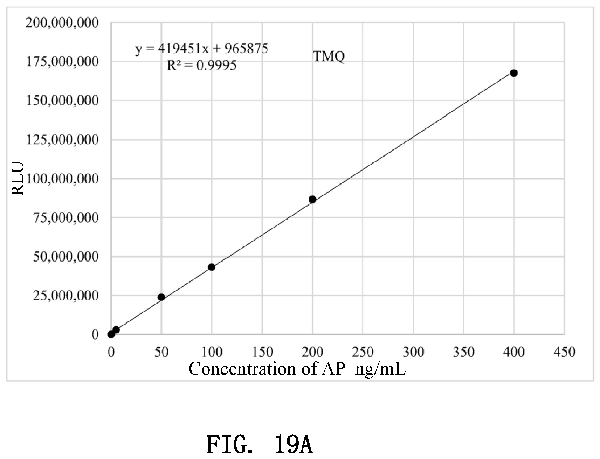

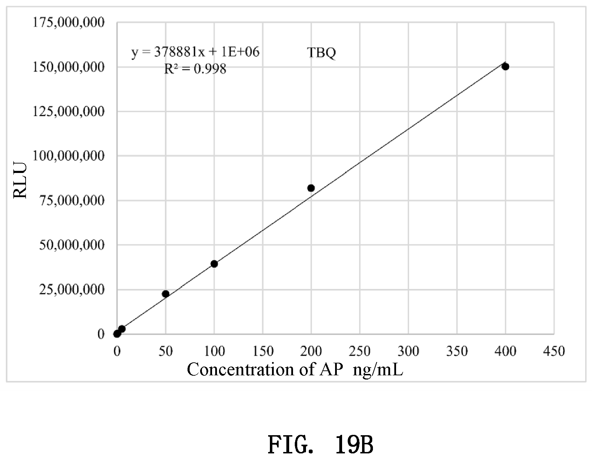

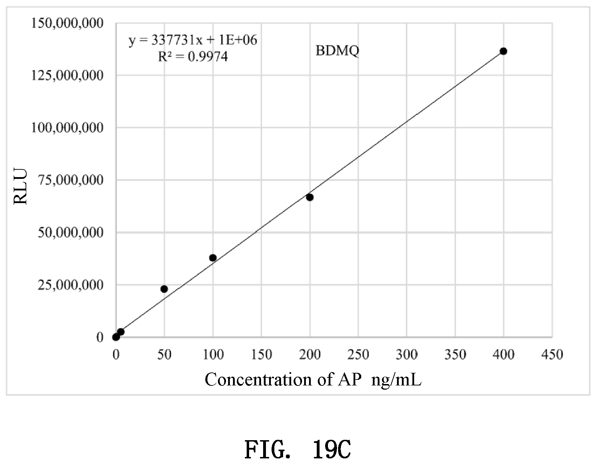

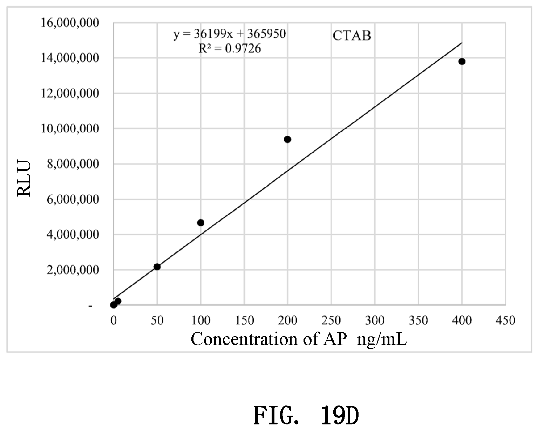

The optical signals (photon counts per second) measured for the chemiluminescent substrate solutions 4-1 to 4-4 were respectively plotted against the concentrations of alkaline phosphatase in the system, and linear fitting was performed, as shown in FIG. 19A to FIG. 19D.

According to the data in Table 8 and FIG. 19A to FIG. 19D, the chemiluminescent substrate solutions 4-1 to 4-3 have low background signals and high luminescence efficiency, and have good linearity (R² ≥ 0.99) within the detected luminescence range. The chemiluminescent substrate solution 4-4 has a high background signal and low luminescence efficiency, cannot maintain good linearity (R² < 0.99) within the detected luminescence range, and also has a small ratio of the optical signal at the upper limit of detection to that at the lower limit of detection (less than 3000).

Example 5: linearity performance using CDP-STAR and different quaternary ammonium salt cationic surfactants
formulation of chemiluminescent substrate solution:
CDP-STAR: 200 mg
fluorescein: 200 mg
quaternary ammonium salt surfactant: 5 g
AMP-HCl: 50 mM
magnesium chloride: 200 mg
sodium azide: 1 g
water: added to a total volume of 1 L

According to the above formulation, wherein the quaternary ammonium salt cationic surfactants are shown in Table 9 below, a series of chemiluminescent substrate solutions 5-1 to 5-4 were prepared.

**Table 9**

| chemiluminescent substrate solution | quaternary ammonium salt cationic surfactant |
|---|---|
| 5-1 | Poly(vinylbenzyltrimethylammonium chloride) (TMQ) |
| 5-2 | poly(vinylbenzyltributylammonium chloride) (TBQ) |
| 5-3 | poly(vinylbenzyl(benzyldimethyl)ammonium chloride) (BDMQ) |
| 5-4 | cetyltrimethylammonium bromide (CTAB) |

The above chemiluminescent substrate solutions 5-1 to 5-4 were tested on a Mindray CL-6000i fully automatic chemiluminescence immunoassay analyser. Alkaline phosphatase solutions with different concentration gradients were respectively mixed with each chemiluminescent substrate solution, and after incubation for a period of time, optical signals were collected, thereby respectively obtaining background signal values (i.e., the alkaline phosphatase concentration was 0) and luminescence signal values under a light collection time of 2 min; the concentrations of alkaline phosphatase in the system and the detection signal values are shown in Table 10 below.

**Table 10**

| chemiluminescent substrate solution | 5-1 | 5-2 | 5-3 | 5-4 |
|---|---|---|---|---|
| surfactant | TMQ | TBQ | BDMQ | CTAB |
| concentration of AP (ng/mL) | RLU | | | |
| 0 | 1,389 | 1,259 | 1,347 | 4,840 |
| 0.03 | 18,728 | 21,879 | 22,814 | 7,395 |
| 0.5 | 283,236 | 288,667 | 324,416 | 28,192 |
| 5 | 3,105,373 | 2,548,347 | 2,807,865 | 227,582 |
| 50 | 21,834,549 | 22,423,689 | 23,834,003 | 2,187,748 |
| 100 | 43,680,543 | 39,859,769 | 39,541,610 | 4,697,443 |
| 200 | 75,720,627 | 70,385,061 | 67,415,324 | 8,562,138 |
| 400 | 131,890,525 | 129,302,663 | 123,413,073 | 12,642,130 |

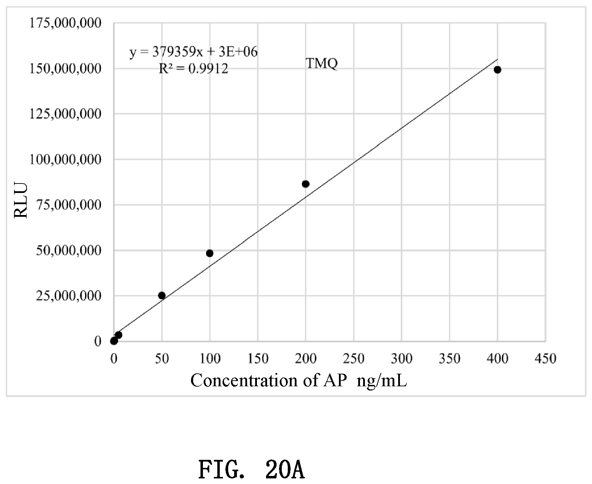

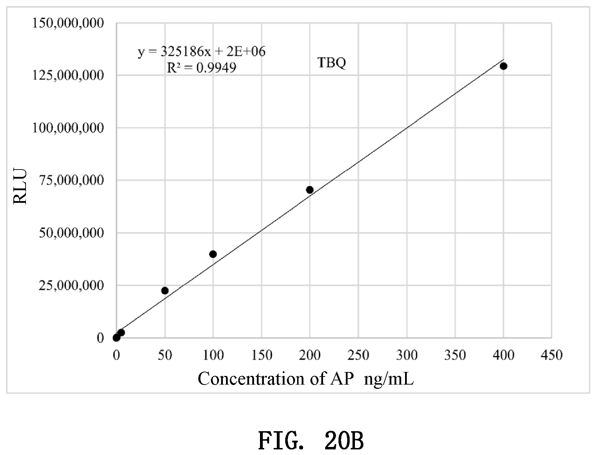

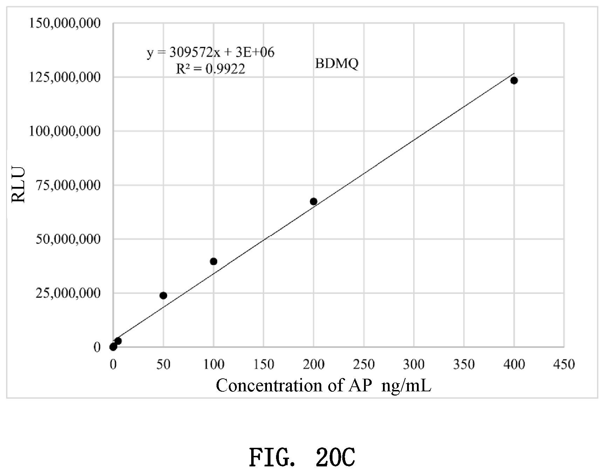

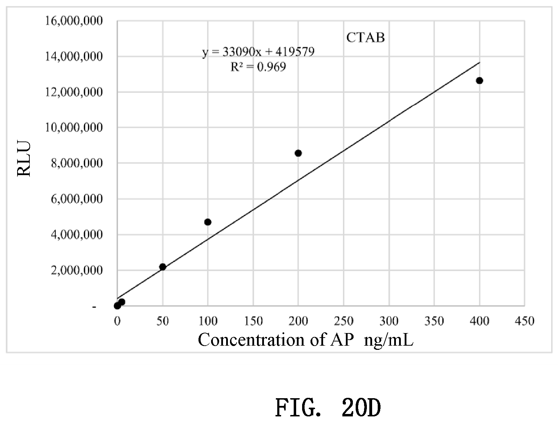

The optical signals (photon counts per second) measured for the chemiluminescent substrate solutions 5-1 to 5-4 were respectively plotted against the concentrations of alkaline phosphatase in the system, and linear fitting was performed, as shown in FIG. 20A to FIG. 20D.

According to the data in Table 10 and FIG. 20A to FIG. 20D, the chemiluminescent substrate solutions 5-1 to 5-3 have low background signals and high luminescence efficiency, and have good linearity (R² ≥ 0.99) within the detected luminescence range. The chemiluminescent substrate solution 5-4 has a high background signal and low luminescence efficiency, cannot maintain good linearity (R² < 0.99) within the detected luminescence range, and also has a small ratio of the optical signal at the upper limit of detection to that at the lower limit of detection (less than 3000).

### Example 6: HCG wide linear detection

A chemiluminescent substrate solution 6-1 was prepared according to the following formulation:
ADP-STAR: 200 mg
fluorescein: 200 mg
Poly(vinylbenzyltrimethylammonium chloride): 5 g
AMP-HCl: 50 mM
magnesium chloride: 200 mg
sodium azide: 1 g
water: added to a total volume of 1 L

A chemiluminescent substrate solution 6-2 was prepared according to the following formulation:
CDP-STAR: 200 mg
fluorescein: 200 mg
Poly(vinylbenzyltrimethylammonium chloride): 5 g
AMP-HCl: 50 mM
magnesium chloride: 200 mg
sodium azide: 1 g
water: added to a total volume of 1 L

Using the above chemiluminescent substrate solutions 6-1 to 6-2, prepared serum samples containing different HCG concentrations were tested on a Mindray CL-6000i fully automatic chemiluminescence immunoassay analyser, wherein the sample concentration range was 1 to 200,000 mIU/mL, and the HCG test reagent kit was a Mindray total β human chorionic gonadotropin (Total β HCG) assay kit (chemiluminescence immunoassay). The photon counts per second of optical signals collected when each sample was incubated with the chemiluminescent substrate solutions 6-1 and 6-2, respectively, for 2 minutes were recorded, and the HCG concentration in the sample was calculated according to the optical signals and recorded as a back-calculated concentration. The relative deviation of the back-calculated concentration was calculated relative to the nominal concentration of HCG in each sample, and the results are shown in Table 11 to Table 12 below.

**Table 11**

| chemiluminescent substrate solution 6-1 | | | | |
|---|---|---|---|---|
| sample number | concentration (mIU/mL) | RLU (photon counts per second) | back-calculated concentration (mIU/mL) | back-calculated deviation (%) |
| 1 | 1.07 | 3,169 | 1.12 | 4.5% |
| 2 | 1.74 | 3,808 | 1.78 | 2.7% |
| 3 | 6.87 | 8,670 | 6.84 | -0.4% |
| 4 | 33.15 | 33,366 | 32.53 | -1.9% |
| 5 | 132.14 | 128,877 | 131.95 | -0.1% |
| 6 | 637.19 | 603,127 | 625.85 | -1.8% |
| 7 | 1316.63 | 1,256,165 | 1306.37 | -0.8% |
| 8 | 3110.32 | 3,083,802 | 3212.81 | 3.3% |
| 9 | 7134.88 | 6,099,186 | 6892.68 | -3.4% |
| 10 | 25346.79 | 22,892,719 | 26507.43 | 4.6% |
| 11 | 121368.48 | 85,727,382 | 117081.93 | -3.5% |
| 12 | 177347.37 | 110,385,488 | 169155.73 | -4.6% |
| 13 | 203006.72 | 130,563,615 | 205715.52 | 1.3% |

**Table 12**

| chemiluminescent substrate solution 6-2 | | | | |
|---|---|---|---|---|
| sample number | concentration (mIU/mL) | RLU (photon counts per second) | back-calculated concentration (mIU/mL) | back-calculated deviation (%) |
| 1 | 1.42 | 2,888 | 1.43 | 0.3% |
| 2 | 2.32 | 3,752 | 2.28 | -1.5% |
| 3 | 7.66 | 9,361 | 7.82 | 2.1% |
| 4 | 47.27 | 50,004 | 47.86 | 1.3% |
| 5 | 217.41 | 217,823 | 212.80 | -2.1% |
| 6 | 1046.56 | 1,041,432 | 1020.40 | -2.5% |
| 7 | 4790.76 | 5,033,768 | 4696.25 | -2.0% |
| 8 | 5495.31 | 5,917,926 | 5634.95 | 2.5% |
| 9 | 23070.90 | 19,067,766 | 23389.04 | 1.4% |
| 10 | 35387.87 | 25,765,982 | 34396.67 | -2.8% |
| 11 | 56101.46 | 37,826,834 | 56620.89 | 0.9% |
| 12 | 212909.72 | 103,479,543 | 213549.24 | 0.3% |

According to Table 11 and Table 12, in the detection of the HCG item, the chemiluminescent substrate solution of the present disclosure can directly perform detection and analysis without dilution when HCG in a sample is within a concentration range of 1 to 200,000 mIU/mL, and deviations of the test results relative to nominal concentrations are all within ±5%.

The description above merely relates to preferred examples of the present disclosure, and is not intended to limit the scope of the disclosure. All equivalent transformations made by using the contents of the specification and the drawings of the disclosure from the concept of the disclosure, or the direct/indirect applications of the contents in other related technical fields are all included in the patent protection scope of the disclosure.

## Claims

1. A chemiluminescence analyser, **characterized in that** the chemiluminescence analyser comprises:
a sample preparation device, configured to cause a blood sample to be tested to be mixed with reaction reagents in a reaction vessel, so that an analyte in the blood sample to be tested reacts with the reaction reagents to obtain a sample solution to be tested, wherein the reaction reagents comprise a capture reagent and a detection reagent labeled with a label;
a substrate supply device, configured to supply a luminescent substrate into the reaction vessel containing the sample solution to be tested, so that the label in the sample solution to be tested undergoes a chemiluminescent reaction with the luminescent substrate;
a photometric device, at least comprising a photometer configured to detect a luminescence signal generated in the chemiluminescent reaction; and
a data processing device, configured to obtain a test result of the analyte in the blood sample to be tested based on the luminescence signal and a preset calibration model;
wherein a first linear detection range of the photometer is [A1, A2] photon counts per second, the first linear detection range represents a range of photon counts outputted by the photometer, and within the first linear detection range, an intensity of the luminescence signal generated in the chemiluminescent reaction is in a linear relationship with photon counts outputted by the photometer, wherein A1 is less than or equal to 2000 and A2 is greater than or equal to 10⁸; a second linear detection range of the luminescent substrate is [B1, B2] photon counts per second, the second linear detection range represents a range of the intensity of the luminescence signal generated in the chemiluminescent reaction, and within the second linear detection range, a number of labels participating in the chemiluminescent reaction is in a linear relationship with the intensity of the luminescence signal generated in the chemiluminescent reaction, wherein B1 is less than or equal to 3000 and B2 is greater than or equal to 10⁸.

2. A chemiluminescence analyser, **characterized in that** the chemiluminescence analyser comprises:
a sample preparation device, configured to cause a blood sample to be tested to be mixed with reaction reagents in a reaction vessel, so that an analyte in the blood sample to be tested reacts with the reaction reagents to obtain a sample solution to be tested, wherein the reaction reagents comprise a capture reagent and a detection reagent labeled with a label;
a substrate supply device, configured to supply a luminescent substrate into the reaction vessel containing the sample solution to be tested, so that the label in the sample solution to be tested undergoes a chemiluminescent reaction with the luminescent substrate;
a photometric device, at least comprising a photometer configured to detect a luminescence signal generated in the chemiluminescent reaction; and
a data processing device, configured to obtain a test result of the analyte in the blood sample to be tested based on the luminescence signal and a preset calibration model;
wherein a first linear detection range of the photometer is [A1, A2] photon counts per second, the first linear detection range represents a range of photon counts outputted by the photometer, and within the first linear detection range, an intensity of the luminescence signal generated in the chemiluminescent reaction is in a linear relationship with photon counts outputted by the photometer, wherein a ratio (A2/A1) of A2 to A1 is greater than or equal to 30000; a second linear detection range of the luminescent substrate is [B1, B2] photon counts per second, the second linear detection range represents a range of the intensity of the luminescence signal generated in the chemiluminescent reaction, and within the second linear detection range, a number of labels participating in the chemiluminescent reaction is in a linear relationship with the intensity of the luminescence signal generated in the chemiluminescent reaction, wherein a ratio (B2/B1) of B2 to B1 is greater than or equal to 30000.

3. The chemiluminescence analyser according to claim 1 or 2, **characterized in that** the lower limit B1 of the second linear detection range is less than or equal to 2000.

4. The chemiluminescence analyser according to any one of claims 1 to 3, **characterized in that** the luminescent substrate is designed such that a background of the luminescent substrate is less than 3000; and/or
the luminescent substrate is designed such that a signal-to-noise ratio of the luminescent substrate is greater than 40000.

5. The chemiluminescence analyser according to any one of claims 1 to 4, **characterized in that** the analyte is human chorionic gonadotropin, wherein the chemiluminescence analyser is designed such that: when a concentration of human chorionic gonadotropin in the blood sample to be tested is greater than or equal to 200000 mIU/ml, the intensity of the luminescence signal generated in the chemiluminescent reaction is less than or equal to a smaller value of A2 and B2.

6. The chemiluminescence analyser according to any one of claims 1 to 5, **characterized in that** the analyte is human chorionic gonadotropin, wherein the chemiluminescence analyser is designed such that: when a concentration of human chorionic gonadotropin in the blood sample to be tested is less than or equal to 0.5 mIU/ml, the intensity (C1) of the luminescence signal generated in the chemiluminescent reaction is greater than or equal to a larger value of A1 and B1.

7. The chemiluminescence analyser according to claim 6, **characterized in that** the chemiluminescence analyser is designed such that: when a concentration of human chorionic gonadotropin in the blood sample to be tested is greater than 0.5 mIU/ml and less than or equal to 5 mIU/ml, preferably less than or equal to 2 mIU/ml, the intensity (C2) of the luminescence signal generated in the chemiluminescent reaction satisfies: wherein a ratio (C2/C1) of C2 to C1 is greater than 2.

8. The chemiluminescence analyser according to any one of claims 1 to 7, **characterized in that** the upper limit B2 of the second linear detection range is greater than the upper limit A2 of the first linear detection range.

9. The chemiluminescence analyser according to any one of claims 1 to 8, **characterized in that** the sample preparation device is further configured to: for each blood sample to be tested containing human chorionic gonadotropin as the analyte, pre-dilute said blood sample to be tested before mixing said blood sample to be tested with the reaction reagents.

10. The chemiluminescence analyser according to claim 9, **characterized in that** the calibration model is obtained by the chemiluminescence analyser using a pre-diluted calibrator.

11. The chemiluminescence analyser according to claim 10, **characterized in that** a pre-dilution factor of the blood sample to be tested is equal to a pre-dilution factor of the calibrator.

12. The chemiluminescence analyser according to any one of claims 1 to 11, **characterized in that** the luminescent substrate comprises a chemiluminescent substrate and a chemiluminescence enhancer, the chemiluminescence enhancer comprises a fluorescent agent and a surfactant, and the fluorescent agent comprises one or more carboxyfluoresceins.

13. The chemiluminescence analyser according to any one of claims 1 to 12, **characterized in that** the luminescent substrate is selected from dioxetane compounds, preferably from one or more of AMPPD, CSPD, and CDP-STAR, and more preferably is CDP-STAR.

14. The chemiluminescence analyser according to any one of claims 1 to 13, **characterized in that** the luminescent substrate is selected from a chlorinated derivative of AMPPD, fluorescein or a carboxy-substituted derivative thereof, and a water-soluble polymeric quaternary ammonium salt cationic surfactant.

15. The chemiluminescence analyser according to claim 14, **characterized in that** the chlorinated derivative of AMPPD is ADP-STAR or CDP-STAR, or
the fluorescein or the carboxy-substituted derivative thereof is fluorescein or 5(6)-carboxyfluorescein, or
the water-soluble polymeric quaternary ammonium salt cationic surfactant is poly(vinylbenzyltrimethylammonium chloride).

16. The chemiluminescence analyser according to any one of claims 1 to 15, **characterized in that** the photometer comprises:
a receiving component, configured to receive an optical signal generated in the chemiluminescent reaction and convert the optical signal into a corresponding electrical signal; and
a processing component, configured to be electrically connected to the receiving component and receive the electrical signal from the receiving component, wherein the processing component comprises a first photon counting module configured to detect a number of pulses of the electrical signal by using a pulse recognition method to obtain a first photon counting result, and a second photon counting module configured to process the electrical signal to obtain a parameter characterizing a number of photons in the optical signal, and obtain a second photon counting result based on the parameter characterizing the number of photons in the optical signal and a preset calibration function, wherein the preset calibration function represents a mapping relationship between the parameter characterizing the number of photons in the optical signal and a photon counting result, and the processing component is further configured to obtain a photon count outputted by the photometer based on the first photon counting result and the second photon counting result, and output the photon count.

17. The chemiluminescence analyser according to claim 16, **characterized in that** the processing component is further configured to:
when the first photon counting result is lower than a first threshold, use the first photon counting result as the photon count outputted by the photometer; and
when the first photon counting result is not lower than the first threshold, use the second photon counting result as the photon count outputted by the photometer.

18. The photometric device according to claim 16 or 17, **characterized in that** the first photon counting module comprises at least one first AD conversion circuit and a first counting circuit electrically connected to each other, the first AD conversion circuit is configured to acquire the electrical signal at a sampling frequency greater than 1 GHz/s and convert the acquired electrical signal into a digital signal and output the digital signal to the first counting circuit, and the first counting circuit is configured to analyse the received digital signal to identify a number of pulses, thereby obtaining the first photon counting result.

19. The chemiluminescence analyser according to any one of claims 16 to 18, **characterized in that** the second photon counting module is configured to process the electrical signal to obtain a direct current component signal, and the parameter characterizing the number of photons in the optical signal comprises a parameter related to the direct current component signal; preferably, the second photon counting module is configured to integrate the electrical signal within a predetermined time period to obtain an integration result, and the parameter characterizing the number of photons in the optical signal comprises a parameter related to the integration result.

20. The photometric device according to claim 19, **characterized in that** the second photon counting module comprises a second AD conversion circuit and a second counting circuit electrically connected to each other, the second AD conversion circuit is configured to convert the electrical signal into a digital signal, and the second counting circuit is configured to process the digital signal to obtain the parameter characterizing the number of photons in the optical signal, and obtain the second photon counting result based on the parameter characterizing the number of photons in the optical signal and the preset calibration function; preferably, the second counting circuit is configured to integrate or sum the digital signal within a predetermined time period to obtain a parameter related to an integration result or a summation result and use said parameter as the parameter characterizing the number of photons in the optical signal.

21. A chemiluminescence analyser, **characterized in that** the chemiluminescence analyser comprises:
a sample preparation device, configured to cause a blood sample to be tested to be mixed with reaction reagents in a reaction vessel, so that an analyte in the blood sample to be tested reacts with the reaction reagents to obtain a sample solution to be tested;
a substrate providing device, configured to supply a luminescent substrate into the reaction vessel containing the sample solution to be tested, so that the sample solution to be tested undergoes a chemiluminescent reaction with the luminescent substrate;
a photometric device, at least comprising a photometer configured to detect a luminescence signal generated in the chemiluminescent reaction; and
a data processing device, configured to obtain a test result of the analyte in the blood sample to be tested based on the luminescence signal and a preset calibration model;
wherein the photometer comprises a receiving component configured to receive the optical signal generated in the chemiluminescent reaction and convert the optical signal into a corresponding electrical signal, and a processing component configured to be electrically connected to the receiving component and receive the electrical signal from the receiving component, wherein the processing component comprises a first photon counting module configured to detect a number of pulses of the electrical signal by using a pulse recognition method to obtain a first photon counting result, and a second photon counting module configured to process the electrical signal to obtain a parameter characterizing a number of photons in the optical signal, and obtain a second photon counting result based on the parameter characterizing the number of photons in the optical signal and a preset calibration function, wherein the calibration function represents a mapping relationship between the parameter characterizing the number of photons in the optical signal and a photon counting result, and the processing component is further configured to obtain a photon count outputted by the photometer based on the first photon counting result and the second photon counting result, and output the photon count; and
a second linear detection range of the luminescent substrate is [B1, B2] photon counts per second, the second linear detection range represents a range of the intensity of the luminescence signal generated in the chemiluminescent reaction, and within the second linear detection range, a number of the labels participating in the chemiluminescent reaction is in a linear relationship with the intensity of the luminescence signal generated in the chemiluminescent reaction, wherein a ratio (B2/B1) of B2 to B1 is greater than or equal to 30000 or B1 is less than or equal to 3000 and B2 is greater than or equal to 10⁸.

22. A chemiluminescence analyser, **characterized in that** the chemiluminescence analyser comprises:
a sample preparation device, configured to cause a blood sample to be tested to be mixed with reaction reagents in a reaction vessel, so that an analyte in the blood sample to be tested reacts with the reaction reagents to obtain a sample solution to be tested;
a substrate providing device, configured to supply a luminescent substrate into the reaction vessel containing the sample solution to be tested, so that the sample solution to be tested undergoes a chemiluminescent reaction with the luminescent substrate;
a photometric device, at least comprising a photometer configured to detect a luminescence signal generated in the chemiluminescent reaction; and
a data processing device, configured to obtain a test result of the analyte in the blood sample to be tested based on the luminescence signal and a preset calibration model;
wherein a first linear detection range of the photometer is [A1, A2] photon counts per second, the first linear detection range represents a range of photon counts output by the photometer, and within the first linear detection range, an intensity of the luminescence signal generated in the chemiluminescent reaction is in a linear relationship with photon counts output by the photometer, wherein a ratio (A2/A1) of A2 to A1 is greater than or equal to 30000 or A1 is less than or equal to 2000 and A2 is greater than or equal to 10⁸, and the luminescent substrate comprises a chemiluminescent substrate and a chemiluminescence enhancer, the chemiluminescence enhancer comprises a fluorescent agent and a surfactant, and the fluorescent agent comprises one or more carboxyfluoresceins.
